# EUROPÄISCHE PATENTSCHRIFT

(11) **EP 1 751 090 B1**
(45) Veröffentlichungstag und Bekanntmachung des Hinweises auf die Patenterteilung: **08.10.2014**
(21) Anmeldenummer: 05741145.6
(22) Anmeldetag: 06.05.2005
(51) Int. Cl.: C07C 271/22, C07C 311/19, C07C 235/14, C07C 237/22

(54) **SÄUREDERIVATE SUBSTITUIERTER CYCLOHEXYL-1,4-DIAMINE**
ACID DERIVATIVES OF SUBSTITUTED CYCLOHEXYL-1,4-DIAMINES
DERIVES D'ACIDE DE CYCLOHEXYLE-1,1-DIAMINES SUBSTITUES

(30) Priorität: 10.05.2004 DE 102004023508
(43) Veröffentlichungstag der Anmeldung: 14.02.2007
(73) Patentinhaber: Grünenthal GmbH, 52078 Aachen (DE)
(72) Erfinder: SUNDERMANN, Corinna, 61381 Friedrichsdorf (DE); SUNDERMANN, Bernd, 61381 Friedrichsdorf (DE)
(74) Vertreter: Bülle, Jan
(86) Internationale Anmeldenummer: PCT/EP2005/004910
(87) Internationale Veröffentlichungsnummer: WO 2005/110973

(56) Entgegenhaltungen:
- WO-A-02/089783

## Beschreibung

Die vorliegende Erfindung betrifft substituierte Cyclohexyl-1,4-diamin-Derivate, Verfahren zu deren Herstellung, Arzneimittel enthaltend diese Verbindungen und die Verwendung von substituierten Cyclohexyl-1,4-diamin-Derivate-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische µ-Opioide wie Morphin sind bei der Therapie starker bis stärkster Schmerzen gut wirksam und von größter Bedeutung für die Schmerztherapie. Es kann jedoch von Vorteil sein, wenn neben dem µ-Opioid-Rezeptor auch andere Opioid-Rezeptoren, insbesondere der ORL-1-Rezeptor, beeinflusst werden, da die reinen µ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application, Wiley VCH, 2002).

Darüber hinaus ist bekannt, dass eine sich eine Beeinflussung der Serotonin- und/oder Noradrenalin-Wiederaufnahme günstig auf das Wirk- und Nebenwirkungsspektrum von Opioiden auswirken kann (Beispiel: Tramadol, s. Opioids with Clinical Relevance: Tramadol, 228-230 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutarat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Aus dem Stand der Technik (WO 02090317) sind strukturell verwandte Verbindungen bekannt, die eine Affinität zum ORL-1-Rezeptor besitzen. Für diese Strukturklasse wurde bislang kein Einfluss auf die Noradrenalin- und Serotonin-Wiederaufnahme beschrieben.

WO 02/089783 beschreibt ebenfalls strukturell vergleichbare Verbindungen, unterscheidet sich jedoch im Substituenten -N(R⁷)(R⁴). In wie weit solche Verbindungen noch pharmakologisch aktiv sind oder sein könnten, ist nicht zu entnehmen.

Aufgabe der vorliegenden Erfindung war es, Arzneimittel zur Verfügung zu stellen, die auf das Opioid-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind. Daüber hinaus sollten die Verbindungen die Noradrenalin- und Serotonin-Wiederaufnahme beeinflussen.

Gegenstand der Erfindung sind daher substituierte Cyclohexyl-1,4-diamin-Derivate-Derivate der allgemeinen Formel I, 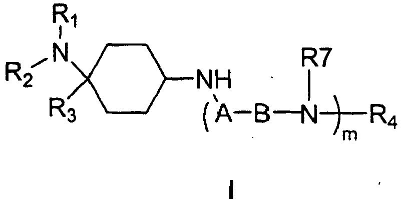 worin
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
   wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
m für 1, 2 oder 3 steht;
A für C(O) oder SO₂ steht;
B für (CR⁵R⁶)ₙ oder Aryl, Heteroaryl oder C₃₋₈-Cycloalkyl, jeweils einfach oder mehrfach substituiert oder unsubstituiert; oder über C₁₋₃-Alkyl, wobei ein einzelnes C-Atom der Alkylkette auch durch O ersetzt sein kann, gebundenes Aryl, C₃₋₈-Cycloalkyl oder Heteroaryl, jeweils einfach oder mehrfach substituiert oder unsubstituiert, wobei die C₁₋₃-Alkylkette den cyclischen Rest mit B und/oder N verknüpfen kann, gegebenenfalls also zwei Alkylketten vorhanden sind; steht; mit n = 1, 2, 3, 4 oder 5;
R⁴ für C(O)R⁹ oder SO₂R⁸ steht;
R⁵, R⁶ für H, C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder. Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R⁷ für H steht oder mit B einen fünf-, sechs- oder siebengliedrigen Ring bildet, der gesättigt oder ungesättigt, aber nicht aromatisch sein kann, und Teil eines polycyclischen Systems sein kann;
R⁸ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R⁹ für OR¹¹, C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R¹¹ für für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, beispielsweise R⁵ und R⁶, sowie der mehrfachen Nennung desselben Substituenten durch den Zusatz eines Zählers, z. B. (CR⁵R⁶)ₙ kann ein Substituent für zwei oder mehrere Reste, beispielsweise R⁵ und/oder R⁶ innerhalb einer Substanz unterschiedliche Bedeutungen annehmen.

Die erfindungsgemäßen Verbindungen zeigen gute Bindung an den µ-Rezeptor und den ORL-1-Rezeptor, aber auch an andere Opioidrezeptoren. Überraschenderweise zeigte es sich, dass die Verbindungen auch gute Inhibitoren der Noradrenalin- und der Serotonin-Wiederaufnahme sind. Damit eignen sie sich auch zur Behandlung von Depressionen, und/oder Bulimie und/oder Anorexie und/oder Katalepsie und/oder zur Anxiolyse und/oder zur Vigilanz- und/oder Libidosteigerung.

Die Ausdrücke "C₁₋₅-Aikyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), 1,1-Dimethylethyl, 1,1-Dimethylpropyl, Butenyl, Butinyl, Pentenyl und Pentinyl umfaßt.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Pyrrolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Isothiazolyl, Imidazolyl, Triazolyl, Triazinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, =O, =S, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NH(C=O)Alkyl, NH(C=O)Aryl), NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkylcycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂₋Aryl, SO₂NH₂, SO₃H, CF₃, =O, =S; -O-CH₂-CH₂-O-; Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen substituierten Cyclohexyl-1,4-diamin-Derivate gilt, dass
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₈-Alkyl, gesättigt oder ungesättigt, verzweigt oder unverzweigt, einfach oder mehrfach substituiert oder unsubstituiert, bedeutet.

Besonders bevorzugt sind substituierte Cyclohexyl-1,4-diamin -Derivate, worin R¹ und R² unabhängig voneinander für CH₃ oder H stehen, wobei R¹ und R² nicht gleichzeitig H bedeuten, oder R¹ und R² für CH₂CH₂OCH₂CH₂, (CH₂)₄, (CH₂)₅ oder (CH₂)₆ stehen.

Weiterhin bevorzugt sind substituierte Cyclohexyl-1,4-diamin -Derivate, worin R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

Besonders bevorzugt sind substituierte Cyclohexyl-1,4-diamin -Derivate, worin R³ für Phenyl, Phenethyl, Thiophenyl, Pyridyl oder Benzyl, jeweils substituiert oder unsubstituiert, steht, besonders bevorzugt für Phenyl, Pyridyl, Thiophenyl, 4-Chlorbenzyl, Benzyl, 3-Fluorphenyl, 3-Chlorbenzyl, 4-Methylbenzyl, 2-Chlorbenzyl, 4-Fluorbenzyl, 3-Methylbenzyl, 2-Methylbenzyl, 3-Fluorbenzyl, 2-Fluorbenzyl oder Phenethyl.

Bevorzugt sind außerdem substituierte Cyclohexyl-1,4-diamin -Derivate, worin B für (CR⁵R⁶)ₙ steht.

Darüber hinaus sind bevorzugt substituierte Cyclohexyl-1,4-diamin -Derivate, worin B für Aryl, Heteroaryl oder C₃₋₈-Cycloalkyl, jeweils einfach oder mehrfach substituiert oder unsubstituiert; oder über C₁₋₃₋Alkyl, wobei ein einzelnes C-Atom der Alkylkette auch durch O ersetzt sein kann, gebundenes Aryl, jeweils einfach oder mehrfach substituiert oder unsubstituiert, wobei die C₁₋₃-Alkylkette den cyclischen Rest mit B und/oder N verknüpfen kann, gegebenenfalls also zwei Alkylketten vorhanden sind; steht.

Weiterhin bevorzugt sind substituierte Cyclohexyl-1,4-diamin -Derivate, worin R⁵ und R⁶ für H, C₁₋₅-Alkyl, jeweils gesättigt oder ungesättigt, verzweigt oder unverzweigt, einfach oder mehrfach substituiert oder unsubstituiert; Aryl, jeweils einfach oder mehrfach substituiert oder unsubstituiert; oder über C₁₋₂-Alkyl gebundenes Aryl oder C₃₋₈-Cycloalkyl, jeweils einfach oder mehrfach substituiert oder unsubstituiert, steht;
Besonders bevorzugt sind Cyclohexyl-1,4-diamin -Derivate, worin R⁵ und R⁶ für H, C₁₋₅-Alkyl, Benzyl oder Phenyl stehen.

Bevorzugt sind auch Cyclohexyl-1,4-diamin -Derivate, worin R⁷ für H steht.

Bevorzugt sind darüber hinaus Cyclohexyl-1,4-diamin -Derivate, worin R⁸ für C₁₋₅-Alkyl, Cyclohexyl, Cyclopentyl, Cyclobutyl, Cycloheptyl, Cyclooctyl, Phenyl, Benzyl, Naphthyl, Thiophenyl, Benzothiophenyl, Furanyl, Pyrazolyl, Benzofuranyl, Benzodioxolanyl, Isochinolinyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenen Phenyl, Naphthyl, Thiophenyl, Benzothiophenyl, Pyridyl, Furyl, Benzofuranyl, Indolyl, Benzodioxanyl, Pyrrolyl, Pyrimidyl oder Pyrazinyl, jeweils unsubstituiert oder einfach oder mehrfach substituiert, steht,
und
R⁹ für OR¹¹, C₁₋₅-Alkyl, Cyclohexyl, Cyclopentyl, Cyclobutyl, Cycloheptyl, Cyclooctyl, Phenyl, Benzyl, Naphthyl, Thiophenyl, Benzothiophenyl, Furanyl. Pyrazolyl, Benzofuranyl, Benzodioxolanyl, Isochinolinyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenen Phenyl, Naphthyl, Thiophenyl, Benzothiophenyl, Pyridyl, Furyl, Benzofuranyl, Indolyl, Benzodioxanyl, Pyrrolyl, Pyrimidyl oder Pyrazinyl, jeweils unsubstituiert oder einfach oder mehrfach substituiert, steht.

Besonders bevorzugt sind substituierte Cyclohexyl-1,4-diamin -Derivate, bei denen R⁸ für Phenyl, C₁₋₅-Alkyl, verzweigt oder unverzweigt, gesättigt oder ungesättigt, oder Benzyl, jeweils unsubstituiert oder einfach oder mehrfach substituiert, steht,
und
R⁹ für OR¹¹, Phenyl, C₁₋₅-Alkyl, verzweigt oder unverzweigt, gesättigt oder ungesättigt, oder Benzyl, jeweils unsubstituiert oder einfach oder mehrfach substituiert, steht.

Darüber hinaus sind substituierte Cyclohexyl-1,4-diamin -Derivate bevorzugt, bei denen R¹¹ für C₁₋₅-Alkyl, Cyclohexyl, CyclopentylCycloheptyl, Cyclooctyl, Phenyl, Benzyl, Naphthyl , Thiophenyl, Benzothiophenyl, Furanyl, Pyrazolyl, Benzofuranyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenes Phenyl, Naphthyl, Thiophenyl, Benzothiophenyl, Pyridyl, Furyl, Benzofuranyl, Indolyl, Pyrrolyl, Pyrimidyl oder Pyrazinyl, jeweils unsubstituiert oder einfach oder mehrfach substituiert, steht,

Besonders bevorzugt sind substituierte Cyclohexyl-1,4-diamin -Derivate, bei denen R¹¹ für Phenyl, C₁₋₅-Alkyl, verzweigt oder unverzweigt, gesättigt oder ungesättigt, Benzyl oder Methylfluorenyl, jeweils unsubstituiert oder einfach oder mehrfach substituiert, steht.

Ganz besonders bevorzugt sind substituierte Cyclohexyl-1,4-diamin-Derivate aus der Gruppe
({1-[4-Dimethylamino-4-(3-fluor-phenyl)-cyclohexylcarbamoyl]-3-methyl-butylcarbamoyl}-methyl)-carbaminsäurebenzylester
{2-[4-Dimethylamino-4-(3-fluor-phenyl)-cyclohexylcarbamoyl]-ethyl}-carbaminsäure-tert-butylester
{[1-((4-Dimethylamino-4-thiophen-2-yl-cyclohexylcarbamoyl)-3-methyl-butylcarbamoyl]-methyl}-carbaminsäurebenzylester
N-(4-Dimethylamino-4-thiophen-2-yl-cyclohexyl)-3-phenyl-2-(tolyl-4-sulfonylamino)-propionamid
[1-((4-Dimethylamino-4-thiophen-2-yl-cyclohexylcarbamoyl)-ethyl]-carbaminsäurebenzylester
N-(4-Dimethylamino-4-pyridin-2-yl-cyclohexyl)-3-phenyl-2-(tolyl-4-sulfonylamino)-propionamid
{[1-((4-Dimethylamino-4-phenyl-cyclohexylcarbamoyl)-3-methyl-butylcarbamoyl]-methyl}-carbaminsäurebenzylester
{[4-Dimethylamino-4-(2-methyl-benzyl)-cyclohexylcarbamoyl]-phenyl-methyl}-carbaminsäurebenzylester
[(4-Azepan-1-yl-4-benzyl-cyclohexylcarbamoyl)-phenyl-methyl]-carbaminsäurebenzylester
{(4-((2-Chlor-benzyl)-4-dimethylamino-cyclohexylcarbamoyl]-phenyl-methyl}-carbaminsäurebenzylester
{1-[4-(2-Chlor-benzyl)-4-dimethylamino-cyclohexylcarbamoyl]-ethyl}-carbaminsäurebenzylester
{[1-((4-Benzyl-4-dimethylamino-cyclohexylcarbamoyl)-3-methyl-butylcarbamoyl]-methyl}-carbaminsäurebenzylester
{1-[4-Dimethylamino-4-(2-methyl-benzyl)-cyclohexylcarbamoyl]-2-phenyl-ethyl}-carbaminsäurebenzylester
({1-[4-Dimethylamino-4-(2-fluor-benzyl)-cyclohexylcarbamoyl]-3-methyl-butylcarbamoyl}-methyl)-carbaminsäurebenzylester
N-{1-[4-Dimethylamino-4-(4-fluor-benzyl)-cyclohexylcarbamoyl]-3-methyl-butyl}-benzamid
{1-[4-(2-Chlor-benzyl)-4-dimethylamino-cyclohexylcarbamoyl]-2-phenyl-ethyl}-carbaminsäure tert-butyl ester
{1-[4-Dimethylamino-4-(2-fluor-benzyl)-cyclohexylcarbamoyl]-2-phenyl-ethyl}-carbaminsäure tert-butyl ester
({1-[4-Dimethylamino-4-(3-methyl-benzyl)-cyclohexylcarbamoyl]-3-methyl-butylcarbamoyl}-methyl)-carbaminsäurebenzylester
({1-[4-Dimethylamino-4-(3-fluor-benzyl)-cyclohexylcarbamoyl]-3-methyl-butylcarbamoyl}-methyl)-carbaminsäurebenzylester
{[1-((4-Dimethylamino-4-phenethyl-cyclohexylcarbamoyl)-3-methyl-butylcarbamoyl]-methyl}-carbaminsäurebenzylester
2-Acetylamino-hexansäure-[4-(2-chlor-benzyl)-4-dimethylamino-cyclohexyl]-amid N-[4-(2-Chlor-benzyl)-4-dimethylamino-cyclohexyl]-3-phenyl-2-(tolyl-4-sulfonylamino)-propionamid
3-tert-Butoxycarbonylamino-N-[4-(2-chlor-benzyl)-4-dimethylamino-cyclohexyl]-bernsteinsäure-cyclohexylester
{1-[4-Dimethylamino-4-(3-methyl-benzyl)-cyclohexylcarbamoyl]-2-phenyl-ethyl}-carbaminsäure tert-butyl ester
({1-[4-Dimethylamino-4-(2-methyl-benzyl)-cyclohexylcarbamoyl]-3-methyl-butylcarbamoyl}-methyl)-carbaminsäurebenzylester
{1-[4-Dimethylamino-4-(2-methyl-benzyl)-cyclohexylcarbamoyl]-ethyl}-carbaminsäurebenzylester
{1-[4-Dimethylamino-4-(2-methyl-benzyl)-cyclohexylcarbamoyl]-2-phenyl-ethyl}-carbaminsäure tert-butyl ester
({1-[4-(3-Chlor-benzyl)-4-dimethylamino-cyclohexylcarbamoyl]-3-methyl-butylcarbamoyl}-methyl)-carbaminsäurebenzylester
({1-[4-Dimethylamino-4-(4-methyl-benzyl)-cyclohexylcarbamoyl]-3-methyl-butylcarbamoyl}-methyl)-carbaminsäurebenzylester
[1-((4-Dimethylamino-4-phenethyl-cyclohexylcarbamoyl)-ethyl]-carbaminsäurebenzylester
{1-[4-Dimethylamino-4-(3-methyl-benzyl)-cyclohexylcarbamoyl]-2-hydroxy-ethyl}-carbaminsäure tert-butyl ester
[5-((4-Benzyl-4-dimethylamino-cyclohexylcarbamoyl)-pentyl]-carbaminsäurebenzylester
{1-[4-(3-Chlor-benzyl)-4-dimethylamino-cyclohexylcarbamoyl]-2-hydroxy-ethyl}-carbaminsäure tert-butyl ester
N-[4-Dimethylamino-4-(2-methyl-benzyl)-cyclohexyl]-3-phenyl-2-(tolyl-4-sulfonylamino)-propionamid
4-Chlor-N-[2-(4-{1-[4-dimethylamino-4-(2-methyl-benzyl)-cyclohexylcarbamoyl]-1-methyl-ethoxy}-phenyl)-ethyl]-benzamid
[1-((4-Benzyl-4-dimethylamino-cyclohexylcarbamoyl)-2-phenyl-ethyl]-carbaminsäure tert-butyl ester
{1-[4-Dimethylamino-4-(3-fluor-benzyl)-cyclohexylcarbamoyl]-2-phenyl-ethyl}-carbaminsäure tert-butyl ester
({1-[4-(4-Chlor-benzyl)-4-dimethylamino-cyclohexylcarbamoyl]-3-methyl-butylcarbamoyl}-methyl)-carbaminsäurebenzylester
{1-[4-Dimethylamino-4-(4-fluor-benzyl)-cyclohexylcarbamoyl]-2-methyl-propyl}-carbaminsäure 9H-fluoren-9-ylmethyl ester
{1-[4-Dimethylamino-4-(3-methyl-benzyl)-cyclohexylcarbamoyl]-ethyl}-carbaminsäurebenzylester
{1-[4-Dimethylamino-4-(2-methyl-benzyl)-cyclohexylcarbamoyl]-2-methyl-propyl}-carbaminsäure 9H-fluoren-9-ylmethyl ester
{1-[4-(3-Chlor-benzyl)-4-dimethylamino-cyclohexylcarbamoyl]-ethyl}-carbaminsäurebenzylester
{[1-((4-Benzyl-4-piperidin-1-yl-cyclohexylcarbamoyl)-3-methyl-butylcarbamoyl]-methyl}-carbaminsäurebenzylester
{5-[4-Dimethylamino-4-(4-methyl-benzyl)-cyclohexylcarbamoyl]-pentyl}-carbaminsäurebenzylester
{[3-Methyl-1-(4-phenyl-4-piperidin-1-yl-cyclohexylcarbamoyl)-butylcarbamoyl]-methyl}-carbaminsäurebenzylester
{1-[4-Dimethylamino-4-(4-methyl-benzyl)-cyclohexylcarbamoyl]-2-hydroxy-ethyl}-carbaminsäure tert-butyl ester
[1-((4-Benzyl-4-dimethylamino-cyclohexylcarbamoyl)-ethyl]-carbaminsäurebenzylester
{1-[4-Dimethylamino-4-(4-methyl-benzyl)-cyclohexylcarbamoyl]-ethyl}-carbaminsäurebenzylester
[1-((4-Benzyl-4-piperidin-1-yl-cyclohexylcarbamoyl)-3-carbamoyl-propyl]-carbaminsäure tert-butyl ester
2-Acetylamino-pent-4-ensäure-[4-(2-chlor-benzyl)-4-dimethylamino-cyclohexyl]-amid
[2-Hydroxy-1-(4-phenyl-4-piperidin-1-yl-cyclohexylcarbamoyl)-ethyl]-carbaminsäure tert-butyl ester
4-Chlor-N-[2-(4-{1-[4-(2-chlor-benzyl)-4-dimethylamino-cyclohexylcarbamoyl]-1-methyl-ethoxy}-phenyl)-ethyl]-benzamid
{3-Carbamoyl-1-[4-(4-chlor-benzyl)-4-dimethylamino-cyclohexylcarbamoyl]-propyl}-carbaminsäure tert-butyl ester
[1-((4-Benzyl-4-piperidin-1-yl-cyclohexylcarbamoyl)-ethyl]-carbaminsäurebenzylester
[2-((4-Dimethylamino-4-phenethyl-cyclohexylcarbamoyl)-ethyl]-carbaminsäure tert-butyl ester
{1-[4-(4-Chlor-benzyl)-4-dimethylamino-cyclohexylcarbamoyl]-ethyl}-carbaminsäurebenzylester
{1-[4-Dimethylamino-4-(4-methyl-benzyl)-cyclohexylcarbamoyl]-2-phenyl-ethyl}-carbaminsäure tert-butyl ester
[1-((4-Morpholin-4-yl-4-phenyl-cyclohexylcarbamoyl)-2-phenyl-ethyl]-carbaminsäure tert-butyl ester
{1-[4-Dimethylamino-4-(3-fluor-benzyl)-cyclohexylcarbamoyl]-ethyl}-carbaminsäurebenzylester
{2-[4-(4-Chlor-benzyl)-4-dimethylamino-cyclohexylcarbamoyl]-ethyl}-carbaminsäure tert-butyl ester
[2-Hydroxy-1-(4-morpholin-4-yl-4-phenyl-cyclohexylcarbamoyl)-ethyl]-carbaminsäure tert-butyl ester
{1-[4-(4-Chlor-benzyl)-4-dimethylamino-cyclohexylcarbamoyl]-2-hydroxy-ethyl}-carbaminsäure tert-butyl ester
{2-[4-Dimethylamino-4-(4-methyl-benzyl)-cyclohexylcarbamoyl]-ethyl}-carbaminsäure tert-butyl ester
{1-[4-(4-Chlor-benzyl)-4-dimethylamino-cyclohexylcarbamoyl]-2-phenyl-ethyl}-carbaminsäure tert-butyl ester
[1-((4-Azepan-1-yl-4-benzyl-cyclohexylcarbamoyl)-2-phenyl-ethyl]-carbaminsäure tert-butyl ester
{2-[4-Dimethylamino-4-(3-fluor-benzyl)-cyclohexylcarbamoyl]-ethyl}-carbaminsäure tert-butyl ester
[1-((4-Morpholin-4-yl-4-phenyl-cyclohexylcarbamoyl)-ethyl]-carbaminsäurebenzylester
[1-((4-Benzyl-4-piperidin-1-yl-cyclohexylcarbamoyl)-2-hydroxy-ethyl]-carbaminsäure tert-butyl ester
2-((2,2,2-Trifluor-acetyl)-1,2,3,4-tetrahydro-isoquinoline-7-sulfonsäure-(4-benzyl-4-pyrrolidin-1-yl-cyclohexyl)-amid
[2-Phenyl-1-(4-phenyl-4-piperidin-1-yl-cyclohexylcarbamoyl)-ethyl]-carbaminsäure tert-butyl ester
{[3-Methyl-1-(4-morpholin-4-yl-4-phenyl-cyclohexylcarbamoyl)-butylcarbamoyl]-methyl}-carbaminsäurebenzylester
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten µ-Opioid-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Cyclohexycarbonsäure-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Cyclohexyl-1,4-diamin-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexyl-1,4-diamin -Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexyl-1,4-diamin -Derivate verzögert freisetzen. Die erfindungsgemäßen substituierten Cyclohexyl-1,4-diamin -Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Cyclohexyl-1,4-diamin-Derivats appliziert.

Für alle vorstehenden Formen der erfindungsgemäßen Arzneimittel ist es besonders bevorzugt, wenn das Arzneimittel neben wenigstens einem substituierten Cyclohexyl-1,4-diamin-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vor.

Der ORL1-Rezeptor, aber auch die anderen Opioid-Rezeptoren, wurden insbesondere im Schmerzgeschehen identifiziert. Entsprechend können erfindungsgemäße substituierte Cyclohexyl-1,4-diamin-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen substituierten Cyclohexyl-1,4-diamin-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Cyclohexyl-1,4-diamin-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vorliegt.

**Ein weiterer Gegenstand der Erfindung ist ein Verfahren zur Herstellung der erfindungsgemäßen substituierten Cyclohexyl-1,4-diamin-Derivate wie in der folgenden Beschreibung und Beispielen ausgeführt.**

Die Reste R⁰¹ und R⁰² haben die für erfindungsgemäße Verbindungen gemäß Formel I für R¹ und R² angegebene Bedeutung und können zusätzlich unabhängig voneinander für eine Schutzgruppe stehen. Die übrigen Reste haben die in Formel I angegebene Bedeutung: 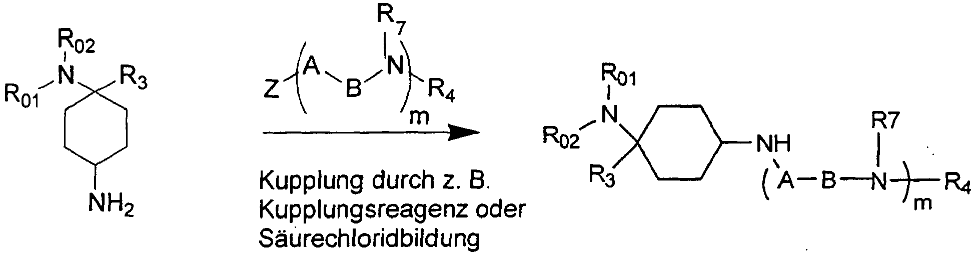 Z = OH, Cl, Aktivester

Zur Darstellung der erfindungsgemäßen Substanzen sind grundsätzlich die vielfältigen, dem Fachmann bekannten Methoden zur Herstellung von Amiden geeignet.
Das erfindungsgemäße Verfahren beruht bevorzugt darauf, substituierte Cyclohexan-1,4-diamine (WO 02090317) mit geeigneten Carbon- oder Sulfonsäuren und/oder Carbon- oder Sulfonsäurederivaten, insbesondere Säurechloriden oder - bromiden, zu verknüpfen und so in erfindungsgemäße Verbindungen zu überführen. Bei Umsetzungen mit Säurechloriden und -bromiden werden polare oder unpolare aprotischen Lösungsmitteln eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base und als Lösungsmittel geeignet. Vorzugsweise werden Säurechloride mit Aminen bei -30 bis +40 °C in Dichlormethan oder Chloroform in Gegenwart von Triethylamin oder Pyridin und ggf. katalytischer Mengen DMAP umgesetzt.
Für die Umsetzung von Carbonsäuren mit einem substituierten Cyclohexan-1,4-diamin (WO 02090317) steht ebenfalls die gesamte Bandbreite der dem Fachmann bekannten Methoden zur Herstellung von Amiden zur Verfügung. Vorteilhaft ist dabei der Einsatz organischer oder anorganischer wasserentziehender Mittel wie z.B. Molsieb, Magnesiumsulfat, Schwefelsäure oder Carbodiimiden wie DCC oder DIC, letztere ggf. in Gegenwart von HOBt. Auch diese Umsetzungen werden vorzugsweise in polaren oder unpolaren aprotischen Lösungsmitteln bei Temperaturen zwischen -30 und +110 °C, bevorzugt -10 und +40 °C durchgeführt. Gegebenenfalls werden anschließend die Schutzgruppen abgespalten.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### Allgemeine Vorschrift:

Zu 0,1 mmol des Cyclohexan-1,4-diamins wurden 0,1 mmol eines Säurechlorids, das aus den entsprechenden Carbonsäuren oder Sulfonsäuren nach dem Fachmann bekannten Methoden hergestellt wurde (s. Tabelle 1), in Gegenwart von 1,05 Equivalenten Triethylamin zugegeben. Es wurde 12 h gerührt und anschließend mit einer 1 M Natriumcarbonatlösung versetzt. Durch Extraktion mit je 3 x 2 ml Dichlormethan und Entfernen des Lösungsmittels wurde das Produkt erhalten.

In Tabelle 1 sind die für den letzten Schritt eingesetzten Säuren für die Beispiele genannt.

**Tabelle 1: Namen der Beispielverbindungen und im letzten Syntheseschritt eingesetzte Carbonsäuren.**

| **Verbindung** | **Eingesetzte Säure** | **Name** |
|---|---|---|
| Beispiel 1 | 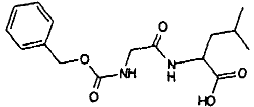 | ({1-[4-Dimethylamino-4-(3-fluor-phenyl)-cyclohexylcarbamoyl]-3-methyl-butylcarbamoyl}-methyl)-carbaminsäurebenzylester |
| Beispiel 2 | 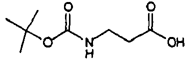 | {2-[4-Dimethylamino-4-(3-fluor-phenyl)-cyclohexylcarbamoyl)-ethyl}-carbaminsäure-tert-butylester |
| Beispiel 3 | 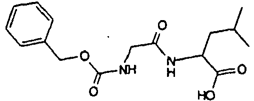 | {[1-(4-Dimethylamino-4-thiophen-2-yl-cyclohexylcarbamoyl)-3-methyl-butylcarbamoyl]-methyl}-carbaminsäurebenzylester |
| Beispiel 4 | 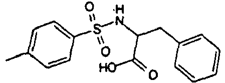 | N-(4-Dimethylamino-4-thiophen-2-yl-cyclohexyl)-3-phenyl-2-(tolyl-4-sulfonylamino)-propionamid |
| Beispiel 5 | 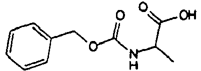 | [1-(4-Dimethylamino-4-thiophen-2-yl-cyclohexylcarbamoyl)-ethyl]-carbaminsäurebenzylester |
| Beispiel 6 | 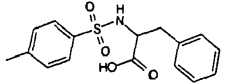 | N-(4-Dimethylamino-4-pyridin-2-yl-cyclohexyl)-3-phenyl-2-(tolyl-4-sulfonylamino)-propionamid |
| Beispiel 7 | 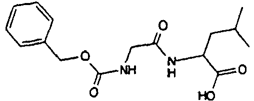 | {[1-(4-Dimethylamino-4-phenyl-cyclohexylcarbamoyl)-3-methyl-butylcarbamoyl]-methyl}-carbaminsäurebenzylester |
| Beispiel 8 | 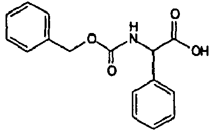 | {[4-Dimethylamino-4-(2-methyl-benzyl)-cyclohexylcarbamoyl]-phenyl-methyl}-carbaminsäurebenzylester |
| Beispiel 9 | 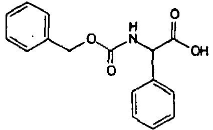 | [(4-Azepan-1-yl-4-benzyl-cyclohexylcarbamoyl)-phenyl-methyl]-carbaminsäurebenzylester |
| Beispiel 10 | 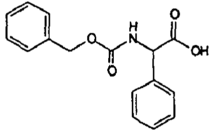 | {[4-(2-Chlor-benzyl)-4-dimethylamino-cyclohexylcarbamoyl]-phenyl-methyl}-carbaminsäurebenzylester |
| Beispiel 11 | 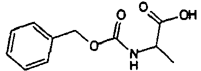 | {1-[4-(2-Chlor-benzyl)-4-dimethylamino-cyclohexylcarbamoyl]-ethyl}-carbaminsäurebenzylester |
| Beispiel 12 | 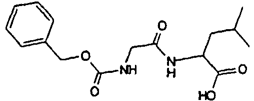 | {[1-(4-Benzyl-4-dimethylamino-cyclohexylcarbamoyl)-3-methyl-butylcarbamoyl]-methyl}-carbaminsäurebenzylester |
| Beispiel 13 | 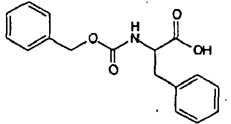 | {1-[4-Dimethylamino-4-(2-methyl-benzyl)-cyclohexylcarbamoyl]-2-phenyl-ethyl}-carbaminsäurebenzylester |
| Beispiel 14 | 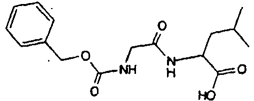 | ({1-[4-Dimethylamino-4-(2-fluor-benzyl)-cyclohexylcarbamoyl]-3-methyl-butylcarbamoyl}-methyl)-carbaminsäurebenzylester |
| Beispiel 15 | 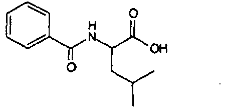 | N-{1-[4-Dimethylamino-4-(4-fluor-benzyl)-cyclohexylcarbamoyl]-3-methyl-butyl}-benzamid |
| Beispiel 16 | 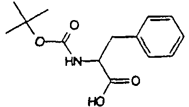 | {1-[4-(2-Chlor-benzyl)-4-dimethylamino-cyclohexylcarbamoyl]-2-phenyl-ethyl}-carbaminsäure tert-butyl ester |
| Beispiel 17 | 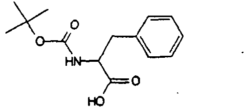 | {1-[4-Dimethylamino-4-(2-fluor-benzyl)-cyclohexylcarbamoyl]-2-phenyl-ethyl}-carbaminsäure tert-butyl ester |
| Beispiel 18 | 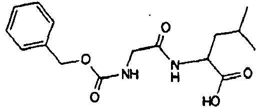 | ({1-[4-Dimethylamino-4-(3-methyl-benzyl)-cyclohexylcarbamoyl]-3-methyl-butylcarbamoyl}-methyl)-carbaminsäurebenzylester |
| Beispiel 19 | 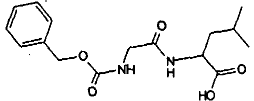 | ({1-[4-Dimethylamino-4-(3-fluor-benzyl)-cyclohexylcarbamoyl]-3-methyl-butylcarbamoyl}-methyl)-carbaminsäurebenzylester |
| Beispiel 20 | 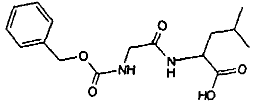 | {[1-(4-Dimethylamino-4-phenethyl-cyclohexylcarbamoyl)-3-methyl-butylcarbamoyl]-methyl}-carbaminsäurebenzylester |
| Beispiel 21 | 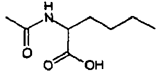 | 2-Acetylamino-hexansäure-[4-(2-chlor-benzyl)-4-dimethylamino-cyclohexyl]-amid |
| Beispiel 22 | 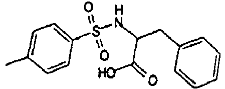 | N-[4-(2-Chlor-benzyl)-4-dimethylamino-cyclohexyl]-3-phenyl-2-(tolyl-4-sulfonylamino)-propionamid |
| Beispiel 23 | 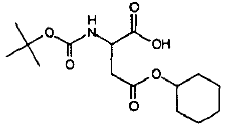 | 3-tert-Butoxycarbonylamino-N-[4-(2-chlor-benzyl)-4-dimethylamino-cyclohexyl]-bernsteinsäure-cyclohexylester |
| Beispiel 24 | 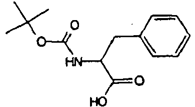 | {1-[4-Dimethylamino-4-(3-methyl-benzyl)-cyclohexylcarbamoyl]-2-phenyl-ethyl}-carbaminsäure tert-butyl ester |
| Beispiel 25 | 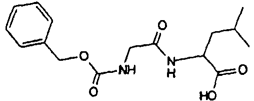 | ({1-[4-Dimethylamino-4-(2-methyl-benzyl)-cyclohexylcarbamoyl]-3-methyl-butylcarbamoyl}-methyl)-carbaminsäurebenzylester |
| Beispiel 26 | 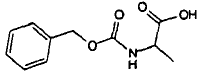 | {1-[4-Dimethylamino-4-(2-methyl-benzyl)-cyclohexylcarbamoyl]-ethyl}-carbaminsäurebenzylester |
| Beispiel 27 | 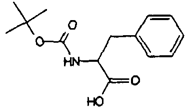 | {1-[4-Dimethylamino-4-(2-methyl-benzyl)-cyclohexylcarbamoyl]-2-phenyl-ethyl}-carbaminsäure tert-butyl ester |
| Beispiel 28 | 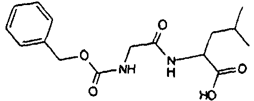 | ({1-[4-(3-Chlor-benzyl)-4-dimethylamino-cyclohexylcarbamoyl]-3-methyl-butylcarbamoyl}-methyl)-carbaminsäurebenzylester |
| Beispiel 29 | 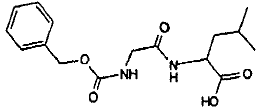 | ({1-[4-Dimethylamino-4-(4-methyl-benzyl)-cyclohexylcarbamoyl]-3-methyl-butylcarbamoyl}-methyl)-carbaminsäurebenzylester |
| Beispiel 30 | 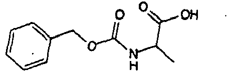 | [1-(4-Dimethylamino-4-phenethyl-cyclohexylcarbamoyl)-ethyl]-carbaminsäurebenzylester |
| Beispiel 31 | 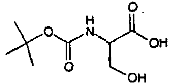 | {1-[4-Dimethylamino-4-(3-methyl-benzyl)-cyclohexylcarbamoyl]-2-hydroxy-ethyl}-carbaminsäure tert-butyl ester |
| Beispiel 32 | 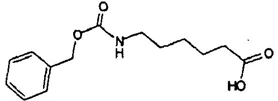 | [5-(4-Benzyl-4-dimethylamino-cyclohexylcarbamoyl)-pentyl]-carbaminsäurebenzylester |
| Beispiel 33 | 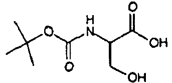 | {1-[4-(3-Chlor-benzyl)-4-dimethylamino-cyclohexylcarbamoyl]-2-hydroxy-ethyl}-carbaminsäure tert-butyl ester |
| Beispiel 34 | 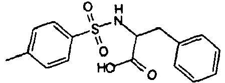 | N-[4-Dimethylamino-4-(2-methyl-benzyl)-cyclohexyl]-3-phenyl-2-(tolyl-4-sulfonylamino)-propionamid |
| Beispiel 35 | 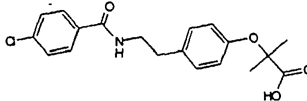 | 4-Chlor-N-[2-(4-{1-[4-dimethylamino-4-(2-methyl-benzyl)-cyclohexylcarbamoyl]-1-methyl-ethoxy}-phenyl)-ethyl]-benzamid |
| Beispiel 36 | 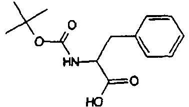 | [1-(4-Benzyl-4-dimethylamino-cyclohexylcarbamoyl)-2-phenyl-ethyl]-carbaminsäure tert-butyl ester |
| Beispiel 37 | 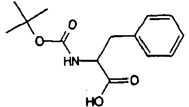 | {1-[4-Dimethylamino-4-(3-fluor-benzyl)-cyclohexylcarbamoyl]-2-phenyl-ethyl}-carbaminsäure tert-butyl ester |
| Beispiel 38 | 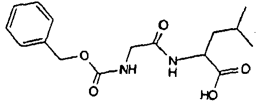 | ({1-[4-(4-Chlor-benzyl)-4-dimethylamino-cyclohexylcarbamoyl]-3-methyl-butylcarbamoyl}-methyl)-carbaminsäurebenzylester |
| Beispiel 39 | 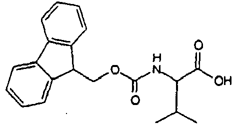 | {1-[4-Dimethylamino-4-(4-fluor-benzyl)-cyclohexylcarbamoyl]-2-methyl-propyl}-carbaminsäure 9H-fluoren-9-ylmethyl ester |
| Beispiel 40 | 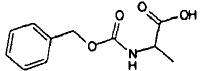 | {1-[4-Dimethylamino-4-(3-methyl-benzyl)-cyclohexylcarbamoyl]-ethyl}-carbaminsäurebenzylester |
| Beispiel 41 | 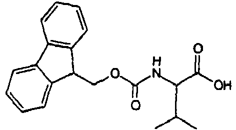 | {1-[4-Dimethylamino-4-(2-methyl-benzyl)-cyclohexylcarbamoyl]-2-methyl-propyl}-carbaminsäure 9H-fluoren-9-ylmethyl ester |
| Beispiel 42 | 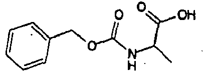 | {1-[4-(3-Chlor-benzyl)-4-dimethylamino-cyclohexylcarbamoyl]-ethyl}-carbaminsäurebenzylester |
| Beispiel 43 | 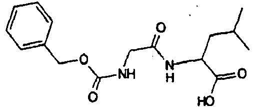 | {[1-(4-Benzyl-4-piperidin-1-yl-cyclohexylcarbamoyl)-3-methyl-butylcarbamoyl]-methyl}-carbaminsäurebenzylester |
| Beispiel 44 | 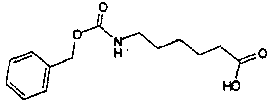 | {5-[4-Dimethylamino-4-(4-methyl-benzyl)-cyclohexylcarbamoyl]-pentyl}-carbaminsäurebenzylester |
| Beispiel 45 | 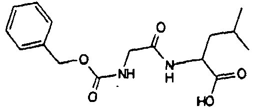 | {[3-Methyl-1-(4-phenyl-4-piperidin-1-yl-cyclohexylcarbamoyl)-butylcarbamoyl]-methyl}-carbaminsäurebenzylester |
| Beispiel 46 | 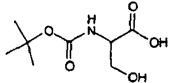 | {1-[4-Dimethylamino-4-(4-methyl-benzyl)-cyclohexylcarbamoyl]-2-hydroxy-ethyl}-carbaminsäure tert-butyl ester |
| Beispiel 47 | 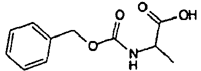 | [1-(4-Benzyl-4-dimethylamino-cyclohexylcarbamoyl)-ethyl]-carbaminsäurebenzylester |
| Beispiel 48 | 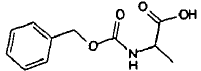 | {1-[4-Dimethylamino-4-(4-methyl-benzyl)-cyclohexylcarbamoyl]-ethyl}-carbaminsäurebenzylester |
| Beispiel 49 | 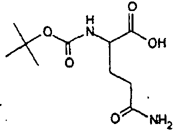 | [1-(4-Benzyl-4-piperidin-1-yl-cyclohexylcarbamoyl)-3-carbamoyl-propyl]-carbaminsäure tert-butyl ester |
| Beispiel 50 | 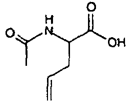 | 2-Acetylamino-pent-4-ensäure-[4-(2-chlor-benzyl)-4-dimethylamino-cyclohexyl]-amid |
| Beispiel 51 | 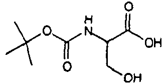 | [2-Hydroxy-1-(4-phenyl-4-piperidin-1-yl-cyclohexylcarbamoyl)-ethyl]-carbaminsäure tert-butyl ester |
| Beispiel 52 | 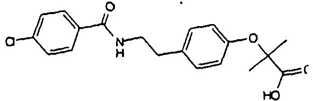 | 4-Chlor-N-[2-(4-{1-[4-(2-chlor-benzyl)-4-dimethylamino-cyclohexylcarbamoyl]-1-methyl-ethoxy}-phenyl)-ethyl]-benzamid |
| Beispiel 53 | 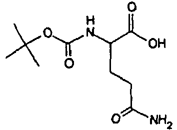 | {3-Carbamoyl-1-[4-(4-chlor-benzyl)-4-dimethylamino-cyclohexylcarbamoyl]-propyl}-carbaminsäure tert-butyl ester |
| Beispiel 54 | 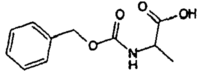 | [1-(4-Benzyl-4-piperidin-1-yl-cyclohexylcarbamoyl)-ethyl]-carbaminsäurebenzylester |
| Beispiel 55 | 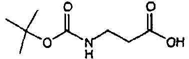 | [2-(4-Dimethylam ino-4-phenethyl-cyclohexylcarbamoyl)-ethyl]-carbaminsäure tert-butyl ester |
| Beispiel 56 | 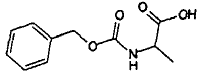 | {1-[4-(4-Chlor-benzyl)-4-dimethylamino-cyclohexylcarbamoyl]-ethyl}-carbaminsäurebenzylester |
| Beispiel 57 | 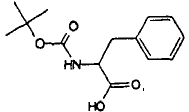 | {1-[4-Dimethylamino-4-(4-methyl-benzyl)-cyclohexylcarbamoyl]-2-phenyl-ethyl}-carbaminsäure tert-butyl ester |
| Beispiel 58 | 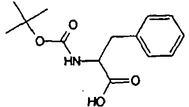 | [1-(4-Morpholin-4-yl-4-phenyl-cyclohexylcarbamoyl)-2-phenyl-ethyl]-carbaminsäure tert-butyl ester |
| Beispiel 59 | 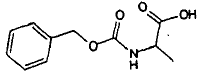 | {1-[4-Dimethylamino-4-(3-fluor-benzyl)-cyclohexylcarbamoyl]-ethyl}-carbaminsäurebenzylester |
| Beispiel 60 | 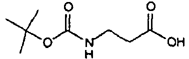 | {2-[4-(4-Chlor-benzyl)-4-dimethylamino-cyclohexylcarbamoyl]-ethyl}-carbaminsäure tert-butyl ester |
| Beispiel 61 | 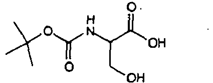 | [2-Hydroxy-1-(4-morpholin-4-yl-4-phenyl-cyclohexylcarbamoyl)-ethyl]-carbaminsäure tert-butyl ester |
| Beispiel 62 | 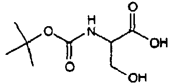 | {1-[4-(4-Chlor-benzyl)-4-dimethylamino-cyclohexylcarbamoyl]-2-hydroxy-ethyl}-carbaminsäure tert-butyl ester |
| Beispiel 63 | 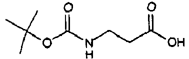 | {2-[4-Dimethylamino-4-(4-methyl-benzyl)-cyclohexylcarbamoyl]-ethyl}-carbaminsäure tert-butyl ester |
| Beispiel 64 | 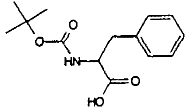 | {1-[4-(4-Chlor-benzyl)-4-dimethylamino-cyclohexylcarbamoyl]-2-phenyl-ethyl}-carbaminsäure tert-butyl ester |
| Beispiel 65 | 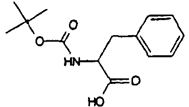 | [1-(4-Azepan-1-yl-4-benzyl-cyclohexylcarbamoyl)-2-phenyl-ethyl]-carbaminsäure tert-butyl ester |
| Beispiel 66 | 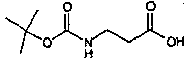 | {2-[4-Dimethylamino-4-(3-fluor-benzyl)-cyclohexylcarbamoyl]-ethyl}-carbaminsäure tert-butyl ester |
| Beispiel 67 | 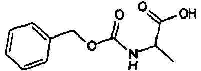 | [1-(4-Morpholin-4-yl-4-phenyl-cyclohexylcarbamoyl)-ethyl]-carbaminsäurebenzylester |
| Beispiel 68 | 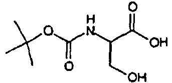 | [1-(4-Benzyl-4-piperidin-1-yl-cyclohexylcarbamoyl)-2-hydroxy-ethyl]-carbaminsäure tert-butyl ester |
| Beispiel 69 | 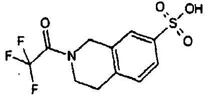 | 2-(2,2,2-Trifluor-acetyl)-1,2,3,4-tetrahydro-isoquinoline-7-sulfonsäure-(4-benzyl-4-pyrrolidin-1-yl-cyclohexyl)-amid |
| Beispiel 70 | 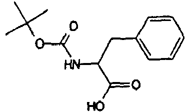 | [2-Phenyl-1-(4-phenyl-4-piperidin-1-yl-cyclohexylcarbamoyl)-ethyl]-carbaminsäure tert-butyl ester |
| Beispiel 71 | 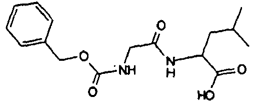 | {[3-Methyl-1-(4-morpholin-4-yl-4-phenyl-cyclohexylcarbamoyl)-butylcarbamoyl]-methyl}-carbaminsäurebenzylester |

### Untersuchungen zur Wirksamkeit der erfindungsgemäßen Verbindungen:

### Messung der ORL1-Bindung

Die Cyclohexan-Derivate der allgemeinen Formel I wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten substituierten Cyclohexyl-1,4-diamin -Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel IIC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

### Messung der Serotonin-Wiederaufnahme

Um diese in vitro Studien durchführen zu können, werden Synäptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### Messung der Noradrenalin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Beispielhaft wurden die folgenden Bindungsdaten bestimmt:

| **Verbindung** | **µ-Opiat-Rezeptor [1µM], % Hemmung** |
|---|---|
| Beispiel 1 | 101 |
| Beispiel 2 | 97 |
| Beispiel 3 | 97 |
| Beispiel 4 | 96 |
| Beispiel 5 | 96 |
| Beispiel 6 | 95 |
| Beispiel 7 | 95 |
| Beispiel 8 | 86 |
| Beispiel 9 | 84 |
| Beispiel 10 | 82 |
| Beispiel 11 | 82 |
| Beispiel 12 | 79 |
| Beispiel 13 | 78 |
| Beispiel 14 | 78 |
| Beispiel 15 | 76 |
| Beispiel 16 | 73 |
| Beispiel 17 | 73 |
| Beispiel 18 | 71 |
| Beispiel 19 | 71 |
| Beispiel 20 | 69 |
| Beispiel 21 | 65 |
| Beispiel 22 | 64 |
| Beispiel 23 | 63 |
| Beispiel 24 | 62 |
| Beispiel 25 | 62 |
| Beispiel 26 | 61 |
| Beispiel 27 | 59 |
| Beispiel 28 | 59 |
| Beispiel 29 | 58 |
| Beispiel 30 | 56 |
| Beispiel 31 | 56 |
| Beispiel 32 | 54 |
| Beispiel 33 | 54 |
| Beispiel 34 | 53 |
| Beispiel 35 | 53 |
| Beispiel 36 | 52 |
| Beispiel 37 | 52 |
| Beispiel 38 | 52 |
| Beispiel 39 | 48 |
| Beispiel 40 | 47 |
| Beispiel 41 | 46 |
| Beispiel 42 | 46 |
| Beispiel 43 | 46 |
| Beispiel 44 | 44 |
| Beispiel 45 | 43 |
| Beispiel 46 | 42 |
| Beispiel 47 | 41 |
| Beispiel 48 | 41 |

| **Verbindung** | **ORL1-Rezeptor [1 µM], % Hemmung** |
|---|---|
| Beispiel 1 | 98 |
| Beispiel 2 | 66 |
| Beispiel 3 | 89 |
| Beispiel 4 | 86 |
| Beispiel 5 | 54 |
| Beispiel 6 | 59 |
| Beispiel 7 | 96 |

### Parenterale Lösung eines erfindungsgemäßen substituierten Cyclohexyl-1,4-diamin-Derivats

38 g eines der erfindungsgemäßen substituierten Cyclohexyl-1,4-diamin-Derivate, hier Beispiel 1, wird in 1 I Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Cyclohexyl-1,4-diamin-Derivate-Derivate der allgemeinen Formel I, 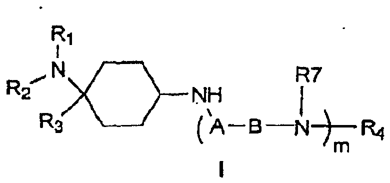 worin
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert öder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
m für 1, 2 oder 3 steht;
A für C(O) oder SO₂ steht;
B für (CR⁵R⁶)ₙ oder Aryl, Heteroaryl oder C₃₋₈-Cycloalkyl, jeweils einfach oder mehrfach substituiert oder unsubstituiert; oder über C₁₋₃-Alkyl, wobei ein einzelnes C-Atom der Alkylkette auch durch O ersetzt sein kann, gebundenes Aryl, C₃₋₈-Cycloalkyl oder Heteroaryl, jeweils einfach oder mehrfach substituiert oder unsubstituiert, wobei die C₁₋₃-Alkylkette den cyclischen Rest mit B und/oder N verknüpfen kann, gegebenenfalls also zwei Alkylketten vorhanden sind; steht; mit n = 1, 2, 3, 4 oder 5;
R⁴ für C(O)R⁹ oder SO₂R⁸ steht;
R⁵, R⁶ für H, C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R⁷ für H steht oder mit B einen fünf-, sechs- oder siebengliedrigen Ring bildet, der gesättigt oder ungesättigt, aber nicht aromatisch sein kann, und Teil eines polycyclischen Systems sein kann;
R⁸ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils
einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
R⁹ für OR¹¹, C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈Cycloalkyl oder Heteroaryl, jeweils einfach oder mehrfach substituiert oder unsubstituiert, steht;
R¹¹ für für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, steht;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte Cyclohexyl-1,4-diamin-Derivate gemäß Anspruch 1, **dadurch gekennzeichnet, dass**
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,

3. Substituierte Cyclohexyl-1,4-diamin-Derivate gemäß Anspruch 1, **dadurch gekennzeichnet, dass** R¹ und R² unabhängig voneinander für CH₃ oder H stehen, wobei R¹ und R² nicht gleichzeitig H bedeuten, oder R¹ und R² für CH₂CH₂OCH₂CH₂, (CH₂)₄, (CH₂)₅ oder (CH₂)₆ stehen.

4. Substituierte Cyclohexyl-1,4-diamin-Derivate gemäß einem der Ansprüche 1-3, **dadurch gekennzeichnet, dass**,
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl,. Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

5. Substituierte Cyclohexyl-1,4-diamin-Derivate gemäß einem der Ansprüche 1-3, **dadurch gekennzeichnet, dass** R³ für Phenyl, Phenethyl, Thiophenyl, Pyridyl oder Benzyl, jeweils substituiert oder unsubstituiert, steht, besonders bevorzugt für Phenyl, Pyridyl, Thiophenyl, 4-Chlorbenzyl, Benzyl, 3-Fluorphenyl, 3-Chlorbenzyl, 4-Methylbenzyl, 2-Chlorbenzyl, 4-Fluorbenzyl, 3-Methylbenzyl, 2-Methylbenzyl, 3-Fluorbenzyl, 2-Fluorbenzyl oder Phenethyl.

6. Substituierte Cyclohexyl-1,4-diamin-Derivate gemäß einem der Ansprüche 1-5, **dadurch gekennzeichnet, dass** B für (CR⁵R⁶)ₙ steht.

7. Substituierte Cyclohexyl-1,4-diamin-Derivate gemäß einem der Ansprüche 1-5, **dadurch gekennzeichnet, dass** B für Aryl, Heteroaryl oder C₃₋₈-Cycloalkyl, jeweils einfach oder mehrfach substituiert oder unsubstituiert; oder über C₁₋₃-Alkyl, wobei ein einzelnes C-Atom der Alkylkette auch durch O ersetzt sein kann, gebundenes Aryl, jeweils einfach oder mehrfach substituiert oder unsubstituiert, wobei die C₁₋₃-Alkylkette den cyclischen Rest mit B und/oder N verknüpfen kann, steht.

8. Substituierte Cyclohexyl-1,4-diamin-Derivate gemäß einem der Ansprüche 1-6, **dadurch gekennzeichnet, dass** R⁵ und R⁶ für H, C₁₋₅-Alkyl, jeweils gesättigt oder ungesättigt, verzweigt oder unverzweigt, einfach oder mehrfach substituiert oder unsubstituiert; Aryl, jeweils einfach oder mehrfach substituiert oder unsubstituiert; oder über C₁₋₂-Alkyl gebundenes Aryl oder C₃₋₈-Cycloalkyl, jeweils einfach oder mehrfach substituiert oder unsubstituiert, steht;

9. Substituierte Cyclohexyl-1,4-diamin-Derivate gemäß einem der Ansprüche 1-6, **dadurch gekennzeichnet, dass** R⁵ und R⁶ für H, C₁₋₅-Alkyl, Benzyl oder Phenyl stehen.

10. Substituierte Cyclohexyl-1,4-diamin-Derivate gemäß einem der Ansprüche 1-9, **dadurch gekennzeichnet, dass** R⁷ für H steht.

11. Substituierte Cyclohexyl-1,4-diamin-Derivate gemäß einem der Ansprüche 1-10, **dadurch gekennzeichnet, dass** R⁸ für C₁₋₅-Alkyl, Cyclohexyl, Cyclopentyl, Cyclobutyl, Cycloheptyl, Cyclooctyl, Phenyl, Benzyl, Naphthyl, Thiophenyl, Benzothiophenyl, Furanyl, Pyrazolyl, Benzofuranyl, Benzodioxolanyl, Isochinolinyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenen Phenyl, Naphthyl, Thiophenyl, Benzothiophenyl, Pyridyl, Furyl, Benzofuranyl, Indolyl, Benzodioxanyl, Pyrrolyl, Pyrimidyl oder Pyrazinyl, jeweils unsubstituiert oder einfach oder mehrfach substituiert, steht,
und
R⁹ für OR¹¹, C₁₋₅-Alkyl, Cyclohexyl, Cyclopentyl, Cyclobutyl, Cycloheptyl, Cyclooctyl, Phenyl, Benzyl, Naphthyl, Thiophenyl, Benzothlophonyl, Furanyl, Pyrazolyl, Benzofuranyl, Benzodloxolanyl, Isochinolinyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenen Phenyl, Naphthyl, Thiophenyl, Benzothiophenyl, Pyridyl, Furyl, Benzofuranyl, Indolyl, Benzodioxanyl, Pyrrolyl, Pyrimidyl oder Pyrazinyl, jeweils unsubstituiert oder einfach oder mehrfach substituiert, steht.

12. Substituierte Cyclohexyl-1,4-diamin-Derivate gemäß einem der Ansprüche 1-1.0, **dadurch gekennzeichnet, dass** R⁸ für Phenyl, C₁₋₅-Alkyl, verzweigt oder unverzweigt, gesättigt oder ungesättigt, oder Benzyl, jeweils unsubstituiert oder einfach oder mehrfach substituiert, steht,
und
R⁹ für OR¹¹, Phenyl, C₁₋₅-Alkyl, verzweigt oder unverzweigt, gesättigt oder ungesättigt, oder Benzyl, jeweils unsubstituiert oder einfach oder mehrfach substituiert, steht.

13. Substituierte Cyclohexyl-1,4-diamin-Derivate gemäß einem der Ansprüche 1-12, **dadurch gekennzeichnet, dass** R¹¹ für C₁₋₅-Alkyl, Cyclohexyl, Cyclopentyl, Cycloheptyl, Cyclooctyl, Phenyl, Benzyl, Naphthyl , Thiophenyl, Benzothiophenyl, Furanyl, Pyrazolyl, Benzofuranyl, Indolyl, Pyrrolyl, Pyridyl, Pyrimidyl oder Pyrazinyl, jeweils unsubstituiert oder einfach oder mehrfach substituiert; über eine gesättigte, unverzweigte substituierte oder unsubstituierte C₁₋₂-Alkyl-Gruppe gebundenen Phenyl, Naphthyl, Thiophenyl, Benzothiophenyl, Pyridyl, Furyl, Benzofuranyl, Indolyl, Pyrrolyl, Pyrimidyl oder Pyrazinyl, jeweils unsubstituiert oder einfach oder mehrfach substituiert, steht,

14. Substituierte Cyclohexyl-1,4-diamin-Derivate gemäß einem der Ansprüche 1-12, **dadurch gekennzeichnet, dass** R¹¹ für Phenyl, C₁₋₅-Alkyl, verzweigt oder unverzweigt, gesättigt oder ungesättigt, Benzyl oder Methylfluorenyl, jeweils unsubstituiert oder einfach oder mehrfach substituiert, steht.

15. Substituierte Cyclohexyl-1,4-diamin-Derivate gemäß einem der Ansprüche 1-14 aus der Gruppe
({1-[4-Dimethylamino-4-(3-fluor-phenyl)-cyclohexylcarbamoyl]-3-methyl-butylcarbamoyl}-methyl)-carbaminsäurebenzylester
{2-[4-Dimethyiamino-4-(3-fluor-phenyl)-cyclohexylcarbamoyl]-ethyl}-carbaminsäure-tert-butylester
{[1-((4-Dimethylamino-4-thiophen-2-yl-cyclohexylcarbamoyl)-3-methyl-butylcarbamoyl]-methyl}-carbaminsäurebenzylester
N-(4-Dimethylamino-4-thiophen-2-yl-cyclohexyl)-3-phenyl-2-(tolyl-4-sulfonylamino)-propionamid
[1-((4-Dimethylamino-4-thiophen-2-yl-cyclohexylcarbamoyl)-ethyl]-carbaminsäurebenzylester
N-(4-Dimethy|amino-4-pyridin-2-yl-cyclohexyl)-3-phenyl-2-(tolyl-4-sulfonylamino)-propionamid
{[1-((4-Dimethylamino-4-phenyl-cyclohexylcarbamoyl)-3-methyl-butylcarbamoyl]-methyl}-carbaminsäurebenzylester
{[4-Dimethylamino-4-(2-methyl-benzyl)-cyclohexylcarbamoyl]-phenyl-methyl}-carbaminsäurebenzylester
[(4-Azepan-1-yl-4-benzyl-cyclohexylcarbamoyl)-phenyl-methyl]-carbaminsäurebenzylester
{[4-((2-Chlor-benzyl)-4-dimethylamino-cyclohexylcarbamoyl]-phenyl-methyl}-carbaminsäurebenzylester
{1-[4-(2-Chlor-benzyl)-4-dimethylamino-cyclohexylcarbamoyl]-ethyl}-carbaminsäurebenzylester
{[1-((4-Benzyl-4-dimethylamino-cyclohexylcarbamoyl)-3-methyl-butylcarbamoyl]-methyl}-carbaminsäurebenzylester
{1-[4-Dimethylamino-4-(2-methyl-benzyl)-cyclohexylcarbamoyl]-2-phenyl-ethyl}-carbaminsäurebenzytester
({1-[4-Dimethylamino-4-(2-fluor-benzyl)-cyclohexylcarbamoyl]-3-methyl-butylcarbamoyl}-methyl)-carbaminsäurebenzylester
N-{1-[4-Dimethylamino-4-(4-fluor-benzyl)-cyclohexylcarbamoyl]-3-methyl-butyl}-benzamid
{1-[4-(2-Chlor-benzyl)-4-dimethylamino-cyclohexylcarbamoyl]-2-phenyl-ethyl}-carbaminsäure tert-butyl ester
{1-[4-Dimethylamino-4-(2-fluor-benzyl)-cyclohexylcarbamoyl]-2-phenyl-ethyl}-carbaminsäure tert-butyl ester
({1-[4-Dimethylamino-4-(3-methyl-benzyl)-cyclohexylcarbamoyl]-3-methyl-butylcarbamoyl}-methyl)-carbaminsäurebenzylester
({1-[4-Dimethylamino-4-(3-fluor-benzyl)-cyclohexylcarbamoyl]-3-methyl-butylcarbamoyl}-methyl)-carbaminsäurebenzylester
{[1-((4-Dimethylamino-4-phenethyl-cyclohexylcarbamoyl)-3-methyl-butylcarbamoyl]-methyl}-carbaminsäurebenzylester
2-Acetylamino-hexansäure-[4-(2-chlor-benzyl)-4-dimethylamino-cyclohexy]-amid N-[4-(2-Chlor-benzyl)-4-dimethylamino-cyclohexyl]-3-phenyl-2-(tolyl-4-sulfonylamino)-propionamid
3-tert-Butoxycarbonylamino-N-[4-(2-chlor-benzyl)-4-dimethylamino-cyclohexyl]-bernsteinsäure-cyclohexylester
{1-[4-Dimethylamino-4-(3-methyl-benzyl)-cyclohexylcarbamoyl]-2-phenyl-ethyl}-carbaminsäure tert-butyl ester
({1-[4-Dimethylamino-4-(2-methyl-benzyl)-cyclohexylcarbamoyl]-3-methyl-butylcarbamoyl}-methyl)-carbamlnsäurebenzylester
{1-[4-Dimethylamino-4-(2-methyl-benzyl)-cyclohexylcarbamoyl]-ethyl}-carbaminsäurebenzylester
{1-[4-Dimethylamino-4-(2-methyl-benzyl)-cyclohexylcarbamoyl]-2-phenyl-ethyl}-carbaminsäure tert-butyl ester
({1-[4-(3-Chlor-benzyl)-4-dimethylamino-cyclohexylcarbamoyl]-3-methyl-butylcarbamoyl}-methyl)-carbaminsäurebenzylester
({1-[4-Dimethylamino-4-(4-methyl-benzyl)-cyclohexylcarbamoyl]-3-methyl-butylcarbamoyl}-methyl)-carbaminsäurebenzylester
[1-((4-Dimethylamino-4-phenethyl-cyclohexylcarbamoyl)-ethyl]-carbaminsäurebenzylester
{1-[4-Dimethylamino-4-(3-methyl-benzyl)-cyclohexylcarbamoyl]-2-hydroxy-ethyl}-carbaminsäure tert-butyl ester
[5-((4-Benzyl-4-dimethylamino-cyclohexylcarbamoyl)-pentyl]-carbaminsäurebenzylester {1-[4-(3-Chlor-benzyl)-4-dimethylamino-cyclohexylcarbamoyl]-2-hydroxy-ethyl}-carbaminsäure tert-butyl ester
N-[4-Dimethylamino-4-(2-methyl-benzyl)-cyclohexyl]-3-phenyl-2-(tolyl-4-sulfonylamino)-propionamid
4-Chlor-N-[2-(4-{1-[4-dimethylamino-4-(2-methyl-benzyl)-cyclohexylcarbamoyl]-1-methyl-ethoxy}-phenyl)-ethyl]-benzamid
[1-((4-Benzyl-4-dimethylamino-cyclohexylcarbamoyl)-2-phenyl-ethyl]-carbaminsäure tert-butyl ester
{1-[4-Dimethylamino-4-(3-fluor-benzyl)-cyclohexylcarbamoyl]-2-phenyl-ethyl}-carbaminsäure tert-butyl ester
({1-[4-(4-Chlor-benzyl)-4-dimethylamino-cyclohexylcarbamoyl]-3-methyl-butylcarbamoyl}-methyl)-carbaminsäurebenzylester
{1-[4-Dimethylamino-4-(4-fluor-benzyl)-cyclohexylcarbamoyl]-2-methyl-propyl}-carbaminsäure 9H-fluoren-9-ylmethyl ester
{1-[4-Dimethylamino-4-(3-methyl-benzyl)-cyclohexylcarbamoyl]-ethyl}-carbaminsäurebenzylester
{1-[4-Dimethylamino-4-(2-methyl-benzyl)-cyclohexylcarbamoyl]-2-methyl-propyl}-carbaminsäure 9H-fluoren-9-ylmethyl ester
{1-[4-(3-Chlor-benzyl)-4-dimethylamino-cyclohexylcarbamoyl]-ethyl}-carbaminsäurebenzylester
{[1-((4-Benzyl-4-piperidin-1-yl-cyclohexylcarbamoyl)-3-methyl-butylcarbamoyl]-methyl}-carbaminsäurebenzylester
{5-[4-Dimethylamino-4-(4-methyl-benzyl)-cyclohexylcarbamoyl]-pentyl}-carbaminsäurebenzylester
{[3-Methyl-1-(4-phenyl-4-piperidin-1-yl-cyclohexylcarbamoyl)-butylcarbamoyl]-methyl}-carbaminsäurebenzylester
{1-[4-Dimethylamino-4-(4-methyl-benzyl)-cyclohexylcarbamoyl]-2-hydroxy-ethyl}-carbaminsäure tert-butyl ester
[1-((4-Benzyl-4-dimethylamino-cyclohexylcarbamoyl)-ethyl]-carbaminsäurebenzylester {1-[4-Dimethylamino-4-(4-methyl-benzyl)-cyclohexylcarbamoyl]-ethyl}-carbaminsäurebenzylester
[1-((4-Benzyl-4-piperidin-1-yl-cyclohexylcarbamoyl)-3-carbamoyl-propyl]-carbaminsäure tert-butyl ester
2-Acetylamino-pent-4-ensäure-[4-(2-chlor-benzyl)-4-dimethylamino-cyclohexyl]-amid [2-Hydroxy-1-(4-phenyl-4-piperidin-1-yl-cyclohexylcarbamoyl)-ethyl]-carbaminsäure tert-butyl ester
4-Chlor-N-[2-(4-{1-[4-(2-chlor-benzyl)-4-dimethylamino-cyclohexylcarbamoyl]-1-methyl-ethoxy}-phenyl)-ethyl]-benzamid
{3-Carbamoyl-1-[4-(4-chlor-benzyl)-4-dimethylamino-cyclohexylcarbamoyl]-propyl}-carbaminsäure tert-butyl ester
[1-((4-Benzyl-4-piperidin-1-yl-cyclohexylcarbamoyl)-ethyl]-carbaminsäurebenzylester
[2-((4-Dimethylamino-4-phenethyl-cyclohexylcarbamoyl)-ethyl]-carbaminsäure tert-butyl ester
{1-[4-(4-Chlor-benzyl)-4-dimethylamino-cyclohexylcarbamoyl]-ethyl}-carbaminsäurebenzylester
{1-[4-Dimethylamino-4-(4-methyl-benzyl)-cyclohexylcarbamoyl]-2-phenyl-ethyl}-carbaminsäure tert-butyl ester
[1-((4-Morpholin-4-yl-4-phenyl-cyclohexylcarbamoyl)-2-phenyl-ethyl]-carbaminsäure tert-butyl ester
{1-[4-Dimethylamino-4-(3-fluor-benzyl)-cyclohexylcarbamoyl]-ethyl}-carbaminsäurebenzylester
{2-[4-(4-Chlor-benzyl)-4-dimethylamino-cyclohexylcarbamoyl]-ethyl}-carbaminsäure tert-butyl ester
[2-Hydroxy-1-(4-morpholin-4-yl-4-phenyl-cyclohexylcarbamoyl)-ethyl]-carbaminsäure tert-butyl ester
{1-[4-(4-Chlor-benzyl)-4-dimethylamino-cyclohexylcarbamoyl]-2-hydroxy-ethyl}-carbaminsäure tert-butyl ester
{2-[4-Dimethylamino-4-(4-methyl-benzyl)-cyclohexylcarbamoyl]-ethyl}-carbaminsäure tert-butyl ester
{1-[4-(4-Chlor-benzyl)-4-dimethylamino-cyclohexylcarbamoyl]-2-phenyl-ethyl}-carbaminsäure tert-butyl ester
[1-((4-Azepan-1-yl-4-benzyl-cyclohexylcarbamoyl)-2-phenyl-ethyl]-carbaminsäure tert-butyl ester
{2-[4-Dimethylamino-4-(3-fluor-benzyl)-cyclohexylcarbamoyl]-ethyl}-carbaminsäure tert-butyl ester
[1-((4-Morpholin-4-yl-4-phenyl-cyclohexylcarbamoyl)-ethyl]-carbaminsäurebenzylester
[1-((4-Benzyl-4-piperidin-1-yl-cyclohexylcarbamoyl)-2-hydroxy-ethyl]-carbaminsäure tert-butyl ester
2-((2,2,2-Trifluor-acetyl)-1,2,3,4-tetrahydro-isoquinoline-7--sulfonsäure-(4-benzyl-4-pyrrolidin-1-yl-cyclohexyl)-amid
[2-Phenyl-1-(4-phenyl-4-piperidin-1-yl-cyclohexylcarbamoyl)-ethyl]-carbaminsäure tert-butyl ester
{[3-Methyl-1-(4-morpholin-4-yl-4-phenyl-cyclohexylcarbamoyl)-butylcarbamoyl]-methyl}-carbaminsäurebenzylester
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

16. Verfahren zur Herstellung von substituierten Cyclohexyl-1,4-diamin-Derivaten gemäß einem der Ansprüche 1-10, **dadurch gekennzeichnet, dass** substituierte Cyclohexan-1,4-diamine mit Carbonsäuren oder Sulfonsäuren der allgemeinen Formel II 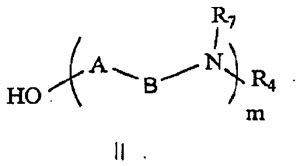 unter Zugabe von Kupplungsreagenzien oder durch Aktivierung der Säurekomponente, insbesondere durch Herstellung des Säurechlorids, verknüpft werden.

17. Arzneimittel enthaltend wenigstens ein substituiertes Cyclohexyl-1,4-diamin-Derivat gemäß einem der Ansprüche 1 bis 15, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

18. Substituierte Cyclohexyl-1,4-diamin-Derivate gemäß einem der Ansprüche 1 bis 15, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Anwendung bei der Behandlung von Schmerz, insbesondere von akutem, neuropathischem oder chronischem Schmerz, zur Anwendung bei der Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, Iokomotorischen Störungen, Diarrhoe, Kachexie, Harninkontinenz bzw. als Muskelrefaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Substituted cyclohexyl 1,4-diamine derivatives-derivatives of the general formula I, 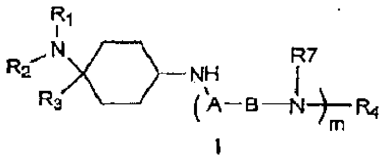 wherein
R¹ and R² independently stand for H; C₁₋₅ alkyl each for saturated or unsaturated, branched or unbranched, mono- or poly-substituted or unsubstituted; C₃₋₈ cycloalkyl, each mono-or poly-substituted or unsubstituted; or for C₁₋₃ alkyl bound aryl, C₃₋₈-cycloalkyl or heteroaryl, each mono-or poly-substituted or unsubstituted;
or the radicals R¹ and R² together stand for CH₂ CH₂O CH₂ CH₂. CH₂ CH₂NR¹⁰ CH₂ CH₂ or (CH₂) ₃₋₆,
whereby R¹⁰ H; C₁₋₅ alkyl means each saturated or unsaturated, branched or unbranched, mono- or poly-substituted or unsubstituted; C₃₋₈-cycloalkyl, each mono-or poly-substituted or unsubstituted; aryl, or heteroaryl each singly or poly-substituted or unsubstituted; or C₁₋₃ alkyl-bound aryl, C₃₋₈ cycloalkyl or heteroaryl, each mono- or poly-substituted or unsubstituted; C (0) phenyl, C (0) heteroaryl, C (0) C₁₋₅ alkyl, substituted or unsubstituted;
R³ stands for substituted or unsubstituted C₁₋₅ alkyl, each saturated or unsaturated, branched or unbranched, mono-or poly-substituted by; C₃₋₈-cycloalkyl, each mono-or poly-substituted or unsubstituted; aryl or heteroaryl, each unsubstituted or mono- or poly-substituted; via C₁₋₃-alkyl group bound aryl, heteroaryl or C₃₋₈-cycloalkyl, each unsubstituted or mono- or poly-substituted;
m stands for 1, 2 or 3;
A stands for C (O) or SO₂ is;
B stands for (CR⁵R⁶)", or aryl, heteroaryl, or C₃₋₈ cycloalkyl each mono-or poly-substituted or unsubstituted; or C₁₋₃-alkyl, wherein a single carbon atom of the alkyl chain may be replaced by O, bound aryl, C₃₋₈-cycloalkyl or heteroaryl, each mono-or poly-substituted or unsubstituted, wherein the C₁₋₃-alkyl chain may connect the cyclic radical B and/or N, that is, two alkyl chains optionally are present; with n = 1, 2, 3, 4 or 5;
R⁴ stands for C (O) R⁹ or SO₂R⁸;
R⁵, R⁶ stands for H, substituted or unsubstituted C₁₋₅ alkyl, each saturated or unsaturated, branched or unbranched, mono-or poly-; each mono-or poly- substituted or unsubstituted C₃₋₈ cycloalkyl; aryl, substituted or unsubstituted or heteroaryl, each mono-or poly-; or C₁₋₃ alkyl bound aryl, C₃₋₈-cycloalkyl or heteroaryl, each mono- or poly-substituted or unsubstituted,
R⁷ stands for H or forms a five-, six-or seven-membered ring B, saturated or unsaturated, but may not be aromatic, and may be part of a polycyclic system;
R⁸ stands for substituted or unsubstituted C₁₋₅ alkyl, each saturated or unsaturated, branched or unbranched, mono-or poly-substituted.; C₃₋₈-cycloalkyl, in each mono- or poly-substituted or unsubstituted; aryl- or heteroaryl, each mono- or poly-substituted or unsubstituted; or via C₁₋₃- alkyl bound aryl, C₃₋₈ cycloalkyl or heteroaryl, each mono-or poly-substituted or unsubstituted;
R⁹ stands for OR¹¹, C₁₋₅ alkyl, each saturated or unsaturated, branched or unbranched, mono-or poly-substituted or unsubstituted; C₃₋₈-cycloalkyl, each mono-or poly-substituted or unsubstituted; aryl-, or heteroaryl, each mono- or poly-substituted or unsubstituted; or C₁₋₃ alkyl-bound aryl, C₃₋₈-cycloalkyl or heteroaryl, each mono- or poly-substituted or unsubstituted;
R¹¹ stands for substituted or unsubstituted C₁₋₅ alkyl, each saturated or unsaturated, branched or unbranched, mono-or poly-substituted; C₃₋₈- cycloalkyl, each mono-or poly-substituted or unsubstituted; aryl- or heteroaryl, each mono- or poly-substituted or unsubstituted; or C₁₋₃ alkyl bound aryl, C₃₋₈-cycloalkyl or heteroaryl, each mono- or poly-substituted or unsubstituted;
in the form of the racemate; of the enantiomers, diastereomers, mixtures of enantiomers or diastereomers or of an individual enantiomer or diastereomer; the bases and/or salts of physiologically acceptable acids or cations.

2. Substituted cyclohexyl 1,4-diamine derivatives according to claim 1, **characterised in that**
R¹ and R² independently of each other stand for H; C₁₋₅-alkyl, saturated or unsaturated, branched or unbranched, mono-or poly-substituted or unsubstituted;
or the radicals R¹ and R² together form a ring and CH₂CH₂OCH₂CH₂, CH₂CH₂NR¹⁰CH₂CH₂ or (CH₂) ₃₋₆.

3. Substituted cyclohexyl 1,4-diamine derivatives according to claim 1, **characterised in that** R¹ and R² independently from each other stand for CH₃ or H, whereby R¹ and R² do not mean simultaneously H, or R¹ and R² stand for CH₂CH₂OCH₂CH₂, (CH₂)₄, (CH₂)₅ or (CH ₂)₆.

4. Substituted cyclohexyl 1,4-diamine derivatives according to one of the claims the 1-3, **characterised in that**,
R³ stands for cyclopentyl, cyclohexyl, phenyl, benzyl, naphthyl, anthracenyl thiophenyl, benzothiophenyl, furyl, benzofuranyl, benzodioxolanyl, indolyl, indanyl, benzodioxanyl, pyrrolyl, pyridyl, pyrimidyl or pyrazinyl, each unsubstituted or mono- or poly-substituted; via a saturated, unbranched C₁₋₂-alkyl group bound C₅₋₆ cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, each unsubstituted or mono- or poly-substituted;
in particular
R³ means phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl, each unsubstituted or mono- or poly-substituted; via a saturated, unbranched C₁₋₂-alkyl group bonded phenyl, furyl or thiophenyl, each unsubstituted or mono-or poly-substituted means.

5. Substituted cyclohexyl 1,4-diamine derivatives according to one of the claims 1-3, **characterised in that** R³ stands for phenyl, phenethyl, thiophenyl, pyridyl or benzyl, each substituted or unsubstituted, is particularly preferred for phenyl, pyridyl, thiophenyl, 4-chlorobenzyl, benzyl, 3-fluorophenyl, 3-chlorobenzyl, 4-methylbenzyl, 2-chlorobenzyl, 4-fluorobenzyl, 3-methylbenzyl, 2-methylenebenzyl, 3 - fluorobenzyl, 2-fluorobenzyl or phenethyl.

6. Substituted cyclohexyl 1,4-diamine derivatives according to one of the claims 1-5, **characterised in that** B stands for (CR⁵R⁶)ₙ.

7. Substituted cyclohexyl 1,4-diamine derivatives according to one of the claims 1-5, wherein B stands for aryl, heteroaryl or C₃₋₈-cycloalkyl, each mono- or poly-substituted or unsubstituted; or C₁₋₃ alkyl, whereby a single carbon atom of the alkyl chain may be replaced by O, bound aryl, each mono-or poly-substituted or unsubstituted, wherein the C₁₋₃-alkyl chain can link to the cyclic radical with B and/or N,

8. Substituted cyclohexyl 1.4-diamine derivatives characterised according to one of the claims. 1-6, wherein R⁵ and R⁶ stand for H, C₁₋₅-alkyl, each saturated or unsaturated, branched or unbranched, mono-or poly-substituted or unsubstituted; aryl, each mono-or poly-substituted or unsubstituted; or C₁₋₂ alkyl or aryl-bound C₃₋₈-cycloalkyl, each mono-or poly-substituted or unsubstituted;

9. Substituted cyclohexyl 1,4-diamine derivatives according to one of the claims 1-6, **characterised in that** R⁵ and R⁶ stand for H, C₁₋₅-alkyl, benzyl or phenyl.

10. Substituted cyclohexyl 1,4-diamine derivatives according to one of the claims 1-9, **characterised in that** R⁷ stands for H.

11. Substituted cyclohexyl 1.4-diamine derivatives according to one of the claims 1-10, **characterised in that** R³ stands for C₁₋₅ alkyl, cyclohexyl, cyclopentyl, cyclobutyl, cycloheptyl, cyclooctyl, phenyl, benzyl, naphthyl, thiophenyl, benzothiophenyl, furanyl pyrazolyl, benzofuranyl, benzodioxolanyl, isoquinolinyl, indolyl, pyrrolyl, pyridyl, pyrimidyl or pyrazinyl, each unsubstituted or mono- or poly-substituted; via a saturated, unbranched substituted or unsubstituted C₁₋₂ alkyl group bonded phenyl, naphthyl, thiophenyl, benzothiophenyl, pyridyl, furyl, benzofuranyl, indolyl, benzodioxanyl, pyrrolyl, pyrimidyl or pyrazinyl, each unsubstituted or mono- or poly-substituted,
and
R⁹ stands for OR¹¹, C₁₋₅-alkyl, cyclohexyl, cyclopentyl, cyclobutyl, cycloheptyl, cyclooctyl, phenyl, benzyl, naphthyl, thiophenyl, benzothiophenyl, furanyl, pyrazolyl, benzofuranyl, benzodioxolanyl, isoquinolinyl, indolyl, pyrrolyl, pyridyl, or pyrazinyl, pyrimidyl each unsubstituted or mono- or poly- substituted; via a saturated, unbranched substituted or unsubstituted C₁₋₂-alkyl group bonded phenyl, naphthyl, thiophenyl, benzothiophenyl, pyridyl, furyl, benzofuranyl, indolyl, benzodioxanyl, pyrrolyl, pyrimidyl or pyrazinyl, each unsubstituted or mono- or poly-substituted.

12. Substituted cyclohexyl 1,4 diamine derivatives according to one of the claims 1-10, **characterised in that** R³ stands for phenyl, C₁₋₅-alkyl, branched or unbranched, saturated or unsaturated, or benzyl, each unsubstituted or mono-or poly-substituted,
and
R⁸ stands for OR¹¹, phenyl, C₁₋₅-alkyl, branched or unbranched, saturated or unsaturated, or benzyl, each unsubstituted or mono- or poly-substituted.

13. Substituted cyclohexyl-1,4-diamine derivatives according to one of the claims 1-12, **characterised in that** R¹¹ stands for C₁₋₅ alkyl, cyclohexyl, cyclopentyl, cycloheptyl, cyclooctyl, phenyl, benzyl, naphthyl, thiophenyl, benzothiophenyl, furanyl, pyrazolyl, benzofuranyl, indolyl, pyrrolyl, pyridyl, pyrimidyl or pyrazinyl, each unsubstituted or mono- or poly-substituted; a saturated, unbranched substituted or unsubstituted C₁₋₂ alkyl group bonded phenyl, naphthyl, thiophenyl, benzothiophenyl, pyridyl, furyl, benzofuranyl, indolyl, pyrrolyl, pyrimidyl or pyrazinyl, each unsubstituted or mono- or poly-substituted,

14. Substituted cyclohexyl 1.4-diamine derivatives according to one of the claims 1-12, **characterised in that** R¹¹ stands for phenyl, C₁₋₅-alkyl, branched or unbranched, saturated or unsaturated, benzyl or methyl-fluorenyl, each unsubstituted or mono- or poly-substituted.

15. Substituted cyclohexyl 1,4-diamine derivatives according to one of the claims 1-14 selected from the group
({1-[4-dimethylamino-4-(3-fluoro-phenylethyl)-cyclohexylcarbamoyl]-3-methyl-butylcarbamoyl}-methyl)-carbamate
{2-[4-dimethylamino-4-(3-fluoro-phenyl)-cyclohexylcarbamoyl]-ethyl}-butyl-carbamic acid tert-butyl ester
{[1 - ((4-Dimethytamino-4-thiophen-2-yl-cyclohexylcarbamoyl)-3-methyl-butylcarbamoyl] - methyl}- carbamic acid benzyl ester
N-(4-Dimethylamino-4-thiophen-2-yl-cyclohexyl)-3-phenyl-2-(tolyl-4-sulfonylamino) - propionamide
[1 - ((4- Dimethylamlno-4-thiophen-2-yl-cyclohexylcarbamoyl)-ethyl] - carbamic acid benzyl ester
N-(4-dimethylamino-4-pyridin-2-yl-cyclohexyl)-3-phenyl-2-(tolyl-4- sulphonylamino)-propionamide
{[1 - ((4-dimethylammonium-4-cyclohexylcarbamoyl-phenyl)-3-methyl-butylcarbamoyl] - methyl}-carbamic acid benzyl ester
{[4-dimethylamino-4-(2-methylbenzyl)-cyclohexylcarbamoyl]-methyl}-phenyl- carbamic acid benzyl ester.
[(4-azepan-1-yl-4∼Benzyl-cyclohexylcarbamoyl) phenyl-methyl] - carbamic {[4 - (2-chlor-benzyl)-4-dimethylamino-cyclohexylcarbamoyl]-phenyl-methyl} -carbamic acid benzyl ester
{1-[4-(2-chlorbenzyl)-4-dimethylamino-cyclohexylcarbamoyl]-ethyl}-carbamic acid benzyl ester
{[1 - ((4-dimethylamino-cyclohexylcarbamoyl)-3-methyl-butylcarbamoyl] - methyl}-carbamic acid benzyl ester
{1 - [4-dimethylamino-4-(2-methyl-benzyl)- cyclohexylcarbamoyl]-2-phenyl-ethyl} -carbamic acid benzyl ester
({1-[4-dimethylamino-4-(2-fluoro-benzyl)-cyclohexylcarbamoyl]-3-methyl-butylcarbamoyl}-methyl}-carbamic acid benzyl ester
N-{1-[4-dimethylamino-4-(4-fluoro-benzyl)-cyclohexylcarbamoyl]-3-methyl-butyl}-benzamide {1-[4-(2-chloro-benzyl)-4-dimethylamino-cyclohexylcarbamoyl]-2-phenyl-ethyl}-carbamic acid tert-butyl ester
{1[4-dimethylamino-4-(2-fluor-benzyl)-cyclohexylcarbamoyl]-2-phenyl-ethyl}-carbamic acid tert-butyl ester
({1-[4-dimethylamino-4-(3-methyl-benzyl)-cyclohexylcarbamoyl]-3-methyl-carbamoyl butyl}-methyl)-carbamic acid benzyl ester
({1-[4-Dimethylamine-4-(3-fluor-benzyl)-cyclohexylcarbamoyl]-3-methyl-butylcarbamoyl}-methyl)-carbamic acid benzyl ester
{[1 - {4-dimethylamino-4-phenethyl-cyclohexylcarbamoyl)-3-methyl-butylcarbannoyl]-methyl)-carbamic acid benzyl ester
2 - acetylamino-hexanoic acid [4-{2-chlor-benzyl)-4-dimethylamino-cyclohexyl]-amide N-{4 -(2-chloro-benzyl)-4-dimethylamino-cydohexyl]-3-phenyl-2-(4-tolyl sulphonylamino)-propionamide
3-tert-buloxycarbonylamino-N-[4 - (2-chloro-benzyl)-4-dimethylamino-cyclohexyl] - succinic acid cyclohexyl ester
{1-[4-dimethylamino-4-(3-methyl-benzyl}cyclohexylcarbamoyl]-2-phenyl-ethyl}-carbamic acid tert-butyl ester
({1-[4-dimethylamino-4-(2-methyl-benzyl)-cyclohexylcarbamoyl]-3-methyl-butylcarbamoyl}-methyl)-carbamic acid benzyl ester
{1-[4-dimethylamino-4-(2-methyl-benzyl)-cyclohexylcarbamoyl]-ethyl}-carbamic acid benzyl ester
{1-[4-dimethylamino-4-(2-methyl-Benzyl)-cyclohexylcarbamoyl]-2-phenyl-ethyl}-carbamic acid tert-butyl ester
((1 - [4 - (3-chloro-benzyl) -4-dimethylamino-cyclohexylcarbamoyl]-3-methyl-butylcarbamoyl}-methyl)-carbammic acid butyl ester
({1-[4-dimethylamino-4-(4-methyl-benzyl)-cyclohexylcarbamoyl]-3-methyl-butylcarbamoyl}-methyl)-carbamate
[1 - ((4-Dimethylamino-4-phenethyl-cyclohexylcarbamoyl)-ethyl] - carbamic acid benzyl ester
{1 - [4-dimethylamino, 4-(3-methylbenzyl)-cyclohexylcarbamoyl]-2-hydroxy-ethyl}-carbamic acid tert-butyl ester
[5 - ((4-benzyl-4-dimethylamino-cyclohexylcarbamoyl)-pentyl]-carbamic acid benzyl ester
{1 - [4 - (3-Chlor-benzyl) '4-dimethylamino-cyclohexylcarbamoyl]-2-hydroxy-ethyl}-carbamic acid tert-butyl ester]
N-[4-dimethylamino-4-(2-methyl-benzyl)-cyclohexyl]-3-phenyl-2-(tolyl-4-sulfonylamino)-propionamide
4-chloro-N-[2 - (4 - {1 - [4-dirnethylamino-4-(2-methyl-benzyl)-cyclohexylcarbamoyl] -1 - methyl-ethoxy}-phenyl)-ethyl-benzamide
[1 - ((4-benzyl-4-dimethylamino-cyclohexylcarbamoyl)-2-phenyl-ethyl]-carbamic acid tert-butyl ester
{1 - [4-dimethylamino-4-(3-fluoro-benzyl)-cyclohexylcarbamoyl]-2-phenyl-ethyl}- carbamic acid tert-butyl ester
({1 - [4 - (4-chloro-benzyl)-4-dimethylamino-cyclohexylcarbamoyl]-3-methyl- butylcarbamoyl}-methyl)-carbamic acid butyl ester
{1 - [4-dimethylamino-4-(4-fluoro-benzyl)-cyclohexylcarbamoyl]-2-methyl-propyl}-carbamic acid 9H-fluoren-9-ylmethyl ester
{1 - [4-dimethylamino-4-{3-methyl-benzyl)-cyclohexylcarbamoyl]-ethyl}-carbamic acid benzyl ester
{1 - [4-dimethylamino-4-(2-methyl-benzyl)-cyclohexylcarbamoyl]-2-methyl propyl}-carbamic acid 9H-fluoren-9-ylmethyl ester
{1 - [4 - (3-chloro-benzyl)-4-dimethylamino-cyclohexylcarbamoyl]-ethyl}-carbamic acid benzyl ester
{[1 - ((4-benzyl-4-piperidin-1-yl-cyclohexylcarbamoyl)-3-methyl-butylcarbamoyl]-methyl}-20carbamic acid butyl ester
{5 - [4-dimethylamino-4-(4-methyl-benzyl)-cyclohexylcarbamoyl]-pentyl}-carbamic acid butyl ester
{[3-methyl-1-(4-phenyl-4-piperidin-1-yl-cyclohexylcarbamoyl)-butylcarbamoyl]-Methyl}-carbamic acid benzyl ester
{1 - [4-dimethylamino-4-(4-methyl-benzyl)-cyclohexylcarbamoyl]-2-hydroxy-ethyl}- carbamic acid tert-butyl ester
[1 - ((4-benzyl-4-dimethylamino-cyclohexylcarbamoyl)-ethyl]-carbamic acid benzyl ester
{1 - [4-dimethylamino-4-{4-methyl-benzyl)-cyclohexylcarbamoyl]-ethyl}-carbamic acid benzyl ester
[1 - ((4-benzyl-4-piperidin-1-yl-cyclohexylcarbamoyl)-3-carbamoylpropyl] carbamic acid tert-butyl ester
2-acetylamino-pent-4-enoic acid [4 - (2-chloro-benzyl)-4-dimethylamino-cydohexyl]-amide [2-hydroxy-1-(4-phenyl-4-piperidin-1-yl-cyclohexylcarbamoyl)-ethyl]-carbamic acid tert-butyl ester
4-chloro-N-[2 - (4 - {1 - [4 - (2-chloro-benzyl)-4-dimethylamino-cyclohexylcarbamoyl]-methyl-1-ethoxy}-phenyl)-ethyl]-benzamide
{3-carbamoyl-1-[4-(4-chlor-benzyl)-4-dimethylamino-cyclohexylcarbamoyl]-propyl}-carbamic acid tert-butyl ester
[1 - ((4-benzyl-4-piperidin-1-yl cyclohexylcarbamoyl)-ethyl-carbamic acid benzyl ester
[2 - ((4-Dimethylamino-4-phenethyl-cyclohexylcarbamoyl)-ethyl]- carbamic acid tert- butyl ester
{1 - [4 (4-chloro-benzyl)-4-dimethylamino-cyclohexylcarbamoyl]-ethyl}-carbamic acid benzyl ester
{1 - [4-dimethylamino-4-(4-methyl-benzyl)-cyclohexylcarbamoyl]-2-phenyl-ethyl}-carbamic acid tert-butyl ester
[1-4-morpholln-4-yl-4-phenyl-cyclohexylparbamoyl)-2-phenyl-ethyl]-carbamic acid butyl ester
{2 - [4-dimethylamino-4-(4-methyl-benzyl)-cyclohexylcarbamoyl]-ethyl}-carbamic acid tert-butyl ester
{1 - [4 - (4-chloro-benzyl)-4-dimethylamino-cyclohexylcarbamoyl]-2-phenyl-ethyl}- carbamic acid tert-butyl ester
[1 - ((4-azepan-1-yl-4-benzyl-cyclohexylcarbamoyl)-2-phenyl-ethyl]-carbamic acid tert-butyl ester
{2 - [4-dimethylamino-4-(3-fluoro-benzyl)-cyclohexylcarbamoyl]-ethyl}-carbamic acid tert-butyl ester
[1 - ((4-morpholin-4-yl-4-phenyl-cyclchexylcarbamoyl) ethyl] carbaminsaurebenzylester
[1 - ((4-benzyl-4-piperidin-1-yl-cyclohexylcarbamoyl)-2-hydroxy-ethyl]-carbamic acid tert-butyl ester
2 - ((2,2,2 -trifluoro-acetyl)-I,4-tetrahydro-2.3 isoquinoline-7-sulfonic acid-(4-benzyl-4-pyrrolidin-1-yl-cyclohexyl)-amide
[2-phenyl-1-(4-phenyl-4-piperidin-1-yl-cyclohexylcarbamoyl)-ethyl]-carbamic acid tert-butyl ester
{[3-methyl-1-(4-morpholin-4-yl-4-phenyl-cyclohexylcarbamoyl)-butylcarbamoyl]-methyl}-carbamic acid benzyl ester in the form of the racemate; of enantiomers, diastereomers, mixtures of enantiomers or diastereomers or of an individual enantiomer or diastereomer; of bases and/or salts of physiologically acceptable acids or cations.

16. Process for the preparation of substituted cyclohexyl-1,4-diamine derivatives according to one of the claims 1-10, **characterised in that** substituted cyclohexane 1,4 diamines with carboxylic acids or sulfonic acids of the general formula II 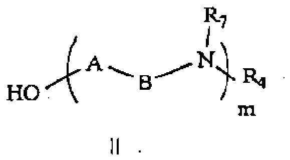 on the addition of coupling reagents or by activating the acid component, in particular by the preparation of the acid chloride, are linked.

17. Medicament containing at least one substituted cyclohexyl 1,4-diamine derivative according to one of the claims 1 to 15, optionally in the form of its racemate, of the pure stereoisomer, in particular enantiomers and diastereomers, in any mixing ratio; in the form of its acids or its bases or in the form of its salts, in particular the physiologically acceptable salts or salts of physiologically acceptable acids or cations; or in the form of its solvates, in particular the hydrates and optionally containing suitable additives and/or auxiliaries and/or optionally other active substances.

18. Substituted cyclohexyl 1,4-diamine derivative according to one of the claims 1 to 15, optionally in the form of its racemate, of the pure stereoisomers, in particular enantiomers and diastereomers, in any mixing ratio; in the form of its acids or its bases or in the form of its salts, in particular the physiologically acceptable salts of salts of physiologically acceptable acids or cations; or in the form of its solvates, in particular hydrates; for the treatment of pain, in particular acute, neuropathic or chronic pain,
For the treatment of anxiety, stress and stress-related syndromes, depression, epilepsy, Alzheimer's disease, senile dementia, catalepsy, general cognitive dysfunction, learning and memory disorders (as a nootropic), withdrawal symptoms, alcohol and/or drug and/or
drug abuse and/or dependency, sexual dysfunctions, cardiovascular diseases, hypotension, hypertension, tinnitus, pruritus, migraine, hearing difficulties, deficient intestinal motility, impaired food intake, anorexia, obesity, locomotor disorders, diarrhoea, cachexia, urinary incontinence or as a muscle relaxant, anticonvulsant or anaesthetic or for co-administration in treatment with an opioid analgesic or with an anaesthetic, for diuresis or antinatriuresis, anxiolysis, for modulation of motor activity, for modulation of neurotransmitter release and treatment of neurodegenerative diseases associated therewith, for treating withdrawal symptoms and/or for reducing the addictive potential of opioids.

## Revendications

1. Dérivés de cyclohexyl-1,4-diamine substitués de formule générale I 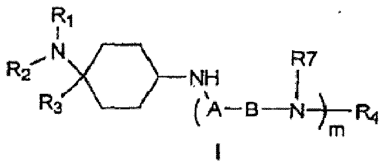 dans laquelle
R¹ et R² représentent indépendamment l'un de l'autre H ; alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
ou les radicaux R¹ et R² représentent ensemble CH₂CH₂OCH₂CH₂, CH₂CH₂NR¹⁰CH₂CH₂ ou (CH₂)₃₋₆,
R¹⁰ signifiant H ; alkyle en C₁₋₅, à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ; C(O)phényle, C(O)hétéroaryle, C(O)alkyle en C₁₋₅, à chaque fois substitué ou non substitué ;
R³ représente alkyle en C₁₋₅, à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois non substitué ou substitué une ou plusieurs fois ; aryle, hétéroaryle ou cycloalkyle en C₃₋₈ relié par un groupe alkyle en C₁₋₃, à chaque fois non substitué ou substitué une ou plusieurs fois ;
m représente 1, 2 ou 3,
A représente C(O) ou SO₂ ;
B représente (CR⁵R⁶)ₙ ou aryle, hétéroaryle ou cycloalkyle en C₃₋₈, à chaque fois substitué une ou plusieurs fois ou non substitué ; ou aryle, cycloalkyle en C₃₋₈ ou hétéroaryle relié par alkyle en C₁₋₃, un atome C individuel de la chaîne alkyle pouvant également être remplacé par O, à chaque fois substitué une ou plusieurs fois ou non substitué, la chaîne alkyle en C₁₋₃ pouvant être reliée au radical cyclique par B et/ou N, deux chaînes alkyle pouvant éventuellement également être présentes ; avec n = 1, 2, 3, 4 ou 5 ;
R⁴ représente C(O)R⁹ ou SO₂R⁸ ;
R⁵, R⁶ représentent H, alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
R⁷ représente H ou forme avec B un cycle à cinq, six ou sept éléments, qui peut être saturé ou insaturé, mais pas aromatique, et qui peut faire partie d'un système polycyclique ; R⁸ représente alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
R⁹ représente OR¹¹, alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
R¹¹ représente alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement acceptables.

2. Dérivés de cyclohexyl-1,4-diamine substitués selon la revendication 1, **caractérisés en ce que**
R¹ et R² représentent indépendamment l'un de l'autre H ; alkyle en C₁₋₅ saturé ou insaturé, ramifié ou non ramifié, substitué une ou plusieurs fois ou non substitué ;
ou les radicaux R¹ et R² forment ensemble un cycle et signifient CH₂CH₂OCH₂CH₂, CH₂CH₂NR¹⁰CH₂CH₂ ou (CH₂)₃₋₆.

3. Dérivés de cyclohexyl-1,4-diamine substitués selon la revendication 1, **caractérisés en ce que** R¹ et R² représentent indépendamment l'un de l'autre CH₃ ou H, R¹ et R² ne signifiant pas simultanément H, ou R¹ et R² représentent CH₂CH₂OCH₂CH₂, (CH₂)₄, (CH₂)₅ ou (CH₂)₆.

4. Dérivés de cyclohexyl-1,4-diamine substitués selon l'une quelconque des revendications 1 à 3, **caractérisés en ce que**
R³ représente cyclopentyle, cyclohexyle, phényle, benzyle, naphtyle, anthracényle, thiophényle, benzothiophényle, furyle, benzofuranyle, benzodioxolanyle, indolyle, indanyle, benzodioxanyle, pyrrolyle, pyridyle, pyrimidyle ou pyrazinyle, à chaque fois non substitué ou substitué une ou plusieurs fois ; cycloalkyle en C₅₋₆, phényle, naphtyle, anthracényle, thiophényle, benzothiophényle, pyridyle, furyle, benzofuranyle, benzodioxolanyle, indolyle, indanyle, benzodioxanyle, pyrrolyle, pyrimidyle ou pyrazinyle relié par un groupe alkyle en C₁₋₂ saturé non ramifié, à chaque fois non substitué ou substitué une ou plusieurs fois ; notamment
R³ signifie phényle, furyle, thiophényle, naphtyle, benzyle, benzofuranyle, indolyle, indanyle, benzodioxanyle, benzodioxolanyle, pyridyle, pyrimidyle, pyrazinyle ou benzothiophényle, à chaque fois non substitué ou substitué une ou plusieurs fois ; phényle, furyle ou thiophényle relié par un groupe alkyle en C₁₋₂ saturé non ramifié, à chaque fois non substitué ou substitué une ou plusieurs fois.

5. Dérivés de cyclohexyl-1,4-diamine substitués selon l'une quelconque des revendications 1 à 3, **caractérisés en ce que** R³ représente phényle, phénéthyle, thiophényle, pyridyle ou benzyle, à chaque fois substitué ou non substitué, de manière particulièrement préférée phényle, pyridyle, thiophényle, 4-chlorobenzyle, benzyle, 3-fluorophényle, 3-chlorobenzyle, 4-méthylbenzyle, 2-chlorobenzyle, 4-fluorobenzyle, 3-méthylbenzyle, 2-méthylbenzyle, 3-fluorobenzyle, 2-fluorobenzyle ou phénéthyle.

6. Dérivés de cyclohexyl-1,4-diamine substitués selon l'une quelconque des revendications 1 à 5, **caractérisés en ce que** B représente (CR⁵R⁶)ₙ.

7. Dérivés de cyclohexyl-1,4-diamine substitués selon l'une quelconque des revendications 1 à 5, **caractérisés en ce que** B représente aryle, hétéroaryle ou cycloalkyle en C₃₋₈, à chaque fois substitué une ou plusieurs fois ou non substitué ; ou aryle relié par alkyle en C₁₋₃, un atome C individuel de la chaîne alkyle pouvant également être remplacé par O, à chaque fois substitué une ou plusieurs fois ou non substitué, la chaîne alkyle en C₁₋₃ pouvant être reliée au radical cyclique par B et/ou N.

8. Dérivés de cyclohexyl-1,4-diamine substitués selon l'une quelconque des revendications 1 à 6, **caractérisés en ce que** R⁵ et R⁶ représentent H, alkyle en C₁₋₅ à chaque fois saturé ou insaturé, ramifié ou non ramifié, substitué une ou plusieurs fois ou non substitué ; aryle, à chaque fois substitué une ou plusieurs fois ou non substitué ; ou aryle ou cycloalkyle en C₃₋₈ relié par alkyle en C₁₋₂, à chaque fois substitué une ou plusieurs fois ou non substitué.

9. Dérivés de cyclohexyl-1,4-diamine substitués selon l'une quelconque des revendications 1 à 6, **caractérisés en ce que** R⁵ et R⁶ représentent H, alkyle en C₁₋₅, benzyle ou phényle.

10. Dérivés de cyclohexyl-1,4-diamine substitués selon l'une quelconque des revendications 1 à 9, **caractérisés en ce que** R⁷ représente H.

11. Dérivés de cyclohexyl-1,4-diamine substitués selon l'une quelconque des revendications 1 à 10, **caractérisés en ce que** R⁸ représente alkyle en C₁₋₅, cyclohexyle, cyclopentyle, cyclobutyle, cycloheptyle, cyclooctyle, phényle, benzyle, naphtyle, thiophényle, benzothiophényle, furanyle, pyrazolyle, benzofuranyle, benzodioxolanyle, isoquinolinyle, indolyle, pyrrolyle, pyridyle, pyrimidyle ou pyrazinyle, à chaque fois non substitué ou substitué une ou plusieurs fois ; phényle, naphtyle, thiophényle, benzothiophényle, pyridyle, furyle, benzofuranyle, indolyle, benzodioxanyle, pyrrolyle, pyrimidyle ou pyrazinyle, relié par un groupe alkyle en C₁₋₂ saturé, non ramifié, substitué ou non substitué, à chaque fois non substitué ou substitué une ou plusieurs fois,
et
R⁹ représente OR¹¹, alkyle en C₁₋₅, cyclohexyle, cyclopentyle, cyclobutyle, cycloheptyle, cyclooctyle, phényle, benzyle, naphtyle, thiophényle, benzothiophényle, furanyle, pyrazolyle, benzofuranyle, benzodioxolanyle, isoquinolinyle, indolyle, pyrrolyle, pyridyle, pyrimidyle ou pyrazinyle, à chaque fois non substitué ou substitué une ou plusieurs fois ; phényle, naphtyle, thiophényle, benzothiophényle, pyridyle, furyle, benzofuranyle, indolyle, benzodioxanyle, pyrrolyle, pyrimidyle ou pyrazinyle, relié par un groupe alkyle en C₁₋₂ saturé, non ramifié, substitué ou non substitué, à chaque fois non substitué ou substitué une ou plusieurs fois.

12. Dérivés de cyclohexyl-1,4-diamine substitués selon l'une quelconque des revendications 1 à 10, **caractérisés en ce que** R⁸ représente phényle, alkyle en C₁₋₅, ramifié ou non ramifié, saturé ou insaturé, ou benzyle, à chaque fois non substitué ou substitué une ou plusieurs fois,
et
R⁹ représente OR¹¹, phényle, alkyle en C₁₋₅, ramifié ou non ramifié, saturé ou insaturé, ou benzyle, à chaque fois non substitué ou substitué une ou plusieurs fois.

13. Dérivés de cyclohexyl-1,4-diamine substitués selon l'une quelconque des revendications 1 à 12, **caractérisés en ce que** R¹¹ représente alkyle en C₁₋₅, cyclohexyle, cyclopentyle, cycloheptyle, cyclooctyle, phényle, benzyle, naphtyle, thiophényle, benzothiophényle, furanyle, pyrazolyle, benzofuranyle, indolyle, pyrrolyle, pyridyle, pyrimidyle ou pyrazinyle, à chaque fois non substitué ou substitué une ou plusieurs fois ; phényle, naphtyle, thiophényle, benzothiophényle, pyridyle, furyle, benzofuranyle, indolyle, pyrrolyle, pyrimidyle ou pyrazinyle, relié par un groupe alkyle en C₁₋₂ saturé, non ramifié, substitué ou non substitué, à chaque fois non substitué ou substitué une ou plusieurs fois.

14. Dérivés de cyclohexyl-1,4-diamine substitués selon l'une quelconque des revendications 1 à 12, **caractérisés en ce que** R¹¹ représente phényle, alkyle en C₁₋₅, ramifié ou non ramifié, saturé ou insaturé, benzyle ou méthylfluorényle, à chaque fois non substitué ou substitué une ou plusieurs fois.

15. Dérivés de cyclohexyl-1,4-diamine substitués selon l'une quelconque des revendications 1 à 14, choisis dans le groupe constitué par :
ester benzylique de l'acide ({1-[4-diméthylamino-4-(3-fluoro-phényl)-cyclohexylcarbamoyl]-3-méthyl-butylcarbamoyl}-méthyl)-carbamique
ester tert-butylique de l'acide {2-[4-diméthylamino-4-(3-fluoro-phényl)-cyclohexylcarbamoyl]-éthyl}-carbamique
ester benzylique de l'acide {[1-(4-diméthylamino-4-thiophén-2-yl-cyclohexylcarbamoyl)-3-méthyl-butylcarbamoyl]-méthyl}-carbamique
N-(4-diméthylamino-4-thiophén-2-yl-cyclohexyl)-3-phényl-2-(tolyl-4-sulfonylamino)-propionamide
ester benzylique de l'acide [1-(4-diméthylamino-4-thiophén-2-yl-cyclohexylcarbamoyl)-éthyl]-carbamique
N-(4-diméthylamino-4-pyridin-2-yl-cyclohexyl)-3-phényl-2-(tolyl-4-sulfonylamino)-propionamide
ester benzylique de l'acide {[1-(4-diméthylamino-4-phényl-cyclohexylcarbamoyl)-3-méthyl-butylcarbamoyl]-méthyl}-carbamique
ester benzylique de l'acide {[4-diméthylamino-4-(2-méthyl-benzyl)-cyclohexylcarbamoyl]-phényl-méthyl}-carbamique
ester benzylique de l'acide [(4-azépan-1-yl-4-benzyl-cyclohexylcarbamoyl)-phényl-méthyl]-carbamique
ester benzylique de l'acide {[4-(2-chloro-benzyl)-4-diméthylamino-cyclohexylcarbamoyl]-phényl-méthyl}-carbamique
ester benzylique de l'acide {1-[4-(2-chloro-benzyl)-4-diméthylamino-cyclohexylcarbamoyl]-éthyl}-carbamique
ester benzylique de l'acide {[1-(4-benzyl-4-diméthylamino-cyclohexylcarbamoyl)-3-méthyl-butylcarbamoyl]-méthyl}-carbamique
ester benzylique de l'acide {1-[4-diméthylamino-4-(2-méthyl-benzyl)-cyclohexylcarbamoyl]-2-phényl-éthyl}-carbamique
ester benzylique de l'acide ({1-[4-diméthylamino-4-(2-fluoro-benzyl)-cyclohexylcarbamoyl]-3-méthyl-butylcarbamoyl}-méthyl)-carbamique
N-{1-[4-diméthylamino-4-(4-fluoro-benzyl)-cyclohexylcarbamoyl]-3-méthyl-butyl}-benzamide ester tert-butylique de l'acide {1-[4-(2-chloro-benzyl)-4-diméthylamino-cyclohexylcarbamoyl]-2-phényl-éthyl}-carbamique
ester tert-butylique de l'acide {1-[4-diméthylamino-4-(2-fluoro-benzyl)-cyclohexylcarbamoyl]-2-phényl-éthyl}-carbamique
ester benzylique de l'acide ({1-[4-diméthylamino-4-(3-méthyl-benzyl)-cyclohexylcarbamoyl]-3-méthyl-butylcarbamoyl}-méthyl)-carbamique
ester benzylique de l'acide ({1-[4-diméthylamino-4-(3-fluoro-benzyl)-cyclohexylcarbamoyl]-3-méthyl-butylcarbamoyl}-méthyl)-carbamique
ester benzylique de l'acide {[1-(4-diméthylamino-4-phénéthyl-cyclohexylcarbamoyl)-3-méthyl-butylcarbamoyl]-méthyl}-carbamique
[4-((2-chloro-benzyl)-4-diméthylamino-cyclohexyl]-amide de l'acide 2-acétylamino-hexanoïque
N-[4-(2-chloro-benzyl)-4-diméthylamino-cyclohexyl]-3-phényl-2-(tolyl-4-sulfonylamino)-propionamide
ester cyclohexylique de l'acide 3-tert-butoxycarbonylamino-N-[4-(2-chloro-benzyl)-4-diméthylamino-cyclohexyl]-succinique
ester tert-butylique de l'acide {1-[4-diméthylamino-4-(3-méthyl-benzyl)-cyclohexylcarbamoyl]-2-phényl-éthyl}-carbamique
ester benzylique de l'acide ({1-[4-diméthylamino-4-(2-méthyl-benzyl)-cyclohexylcarbamoyl]-3-méthyl-butylcarbamoyl}-méthyl)-carbamique
ester benzylique de l'acide {1-[4-diméthylamino-4-(2-méthyl-benzyl)-cyclohexylcarbamoyl]-éthyl}-carbamique
ester tert-butylique de l'acide {1-[4-diméthylamino-4-(2-méthyl-benzyl)-cyclohexylcarbamoyl]-2-phényl-éthyl}-carbamique
ester benzylique de l'acide ({1-[4-(3-chloro-benzyl)-4-diméthylamino-cyclohexylcarbamoyl]-3-méthyl-butylcarbamoyl}-méthyl)-carbamique
ester benzylique de l'acide ({1-[4-diméthylamino-4-(4-méthyl-benzyl)-cyclohexylcarbamoyl]-3-méthyl-butylcarbamoyl}-méthyl)-carbamique
ester benzylique de l'acide [1-(4-diméthylamino-4-phénéthyl-cyclohexylcarbamoyl)-éthyl]-carbamique
ester tert-butylique de l'acide {1-[4-diméthylamino-4-(3-méthyl-benzyl)-cyclohexylcarbamoyl]-2-hydroxy-éthyl}-carbamique
ester benzylique de l'acide [5-(4-benzyl-4-diméthylamino-cyclohexylcarbamoyl)-pentyl]-carbamique
ester tert-butylique de l'acide {1-[4-(3-chloro-benzyl)-4-diméthylamino-cyclohexylcarbamoyl]-2-hydroxy-éthyl}-carbamique
N-[4-diméthylamino-4-(2-méthyl-benzyl)-cyclohexyl]-3-phényl-2-(tolyl-4-sulfonylamino)-propionamide
4-chloro-N-[2-(4-{1-[4-diméthylamino-4-(2-méthyl-benzyl)-cyclohexylcarbamoyl]-1-méthyl-éthoxy}-phényl)-éthyl]-benzamide
ester tert-butylique de l'acide [1-(4-benzyl-4-diméthylamino-cyclohexylcarbamoyl)-2-phényl-éthyl]-carbamique
ester tert-butylique de l'acide {1-[4-diméthylamino-4-(3-fluoro-benzyl)-cyclohexylcarbamoyl]-2-phényl-éthyl}-carbamique
ester benzylique de l'acide ({1-[4-(4-chloro-benzyl)-4-diméthylamino-cyclohexylcarbamoyl]-3-méthyl-butylcarbamoyl}-méthyl)-carbamique
ester 9H-fluorén-9-ylméthylique de l'acide {1-[4-diméthylamino-4-(4-fluoro-benzyl)-cyclohexylcarbamoyl]-2-méthyl-propyl}-carbamique
ester benzylique de l'acide {1-[4-diméthylamino-4-(3-méthyl-benzyl)-cyclohexylcarbamoyl]-éthyl}-carbamique
ester 9H-fluorén-9-ylméthylique de l'acide {1-[4-diméthylamino-4-(2-méthyl-benzyl)-cyclohexylcarbamoyl]-2-méthyl-propyl}-carbamique
ester benzylique de l'acide {1-[4-(3-chloro-benzyl)-4-diméthylamino-cyclohexylcarbamoyl]-éthyl}-carbamique
ester benzylique de l'acide {[1-(4-benzyl-4-pipéridin-1-yl-cyclohexylcarbamoyl)-3-méthyl-butylcarbamoyl]-méthyl}-carbamique
ester benzylique de l'acide {5-[4-diméthylamino-4-{4-méthyl-benzyl)-cyclohexylcarbamoyl]-pentyl}-carbamique
ester benzylique de l'acide {[3-méthyl-1-(4-phényl-4-pipéridin-1-yl-cyclohexylcarbamoyl)-butylcarbamoyl]-méthyl}-carbamique
ester tert-butylique de l'acide {1-[4-diméthylamino-4-(4-méthyl-benzyl)-cyclohexylcarbamoyl]-2-hydroxy-éthyl}-carbamique
ester benzylique de l'acide [1-(4-benzyl-4-diméthylamino-cyclohexylcarbamoyl)-éthyl]-carbamique
ester benzylique de l'acide {1-[4-diméthylamino-4-(4-méthyl-benzyl)-cyclohexylcarbamoyl]-éthyl}-carbamique
ester tert-butylique de l'acide [1-(4-benzyl-4-pipéridin-1-yl-cyclohexylcarbamoyl)-3-carbamoyl-propyl]-carbamique
[4-((2-chloro-benzyl)-4-diméthylamino-cyclohexyl]-amide de l'acide 2-acétylamino-pent-4-énoïque
ester tert-butylique de l'acide [2-hydroxy-1-(4-phényl-4-pipéridin-1-yl-cyclohexylcarbamoyl)-éthyl]-carbamique
4-chloro-N-[2-(4-{1-[4-(2-chloro-benzyl)-4-diméthylamino-cyclohexylcarbamoyl]-1-méthyl-éthoxy}-phényl)-éthyl]-benzamide
ester tert-butylique de l'acide {3-carbamoyl-1-[4-(4-chloro-benzyl)-4-diméthylamino-cyclohexylcarbamoyl]-propyl}-carbamique
ester benzylique de l'acide [1-(4-benzyl-4-pipéridin-1-yl-cyclohexylcarbamoyl)-éthyl]-carbamique
ester tert-butylique de l'acide [2-(4-diméthylamino-4-phénéthyl-cyclohexylcarbamoyl)-éthyl]-carbamique
ester benzylique de l'acide {1-[4-(4-chloro-benzyl)-4-diméthylamino-cyclohexylcarbamoyl]-éthyl}-carbamique
ester tert-butylique de l'acide {1-[4-diméthylamino-4-(4-méthyl-benzyl)-cyclohexylcarbamoyl]-2-phényl-éthyl}-carbamique
ester tert-butylique de l'acide [1-(4-morpholin-4-yl-4-phényl-cyclohexylcarbamoyl)-2-phényl-éthyl]-carbamique
ester benzylique de l'acide {1-[4-diméthylamino-4-(3-fluoro-benzyl)-cyclohexylcarbamoyl]-éthyl}-carbamique
ester tert-butylique de l'acide {2-[4-(4-chloro-benzyl)-4-diméthylamino-cyclohexylcarbamoyl]-éthyl}-carbamique
ester tert-butylique de l'acide [2-hydroxy-1-(4-morpholin-4-yl-4-phényl-cyclohexylcarbamoyl)-éthyl]-carbamique
ester tert-butylique de l'acide {1-[4-(4-chloro-benzyl)-4-diméthylamino-cyclohexylcarbamoyl]-2-hydroxy-éthyl}-carbamique
ester tert-butylique de l'acide {2-[4-diméthylamino-4-(4-méthyl-benzyl)-cyclohexylcarbamoyl]-éthyl}-carbamique
ester tert-butylique de l'acide {1-[4-(4-chloro-benzyl)-4-diméthylamino-cyclohexylcarbamoyl]-2-phényl-éthyl}-carbamique
ester tert-butylique de l'acide [1-(4-azépan-1-yl-4-benzyl-cyclohexylcarbamoyl)-2-phényl-éthyl]-carbamique
ester tert-butylique de l'acide {2-[4-diméthylamino-4-(3-fluoro-benzyl)-cyclohexylcarbamoyl]-éthyl}-carbamique
ester benzylique de l'acide [1-(4-morpholin-4-yl-4-phényl-cyclohexylcarbamoyl)-éthyl]-carbamique
ester tert-butylique de l'acide [1-(4-benzyl-4-pipéridin-1-yl-cyclohexylcarbamoyl)-2-hydroxy-éthyl]-carbamique
(4-benzyl-4-pyrrolidin-1-yl-cyclohexyl)-amide de l'acide 2-(2,2,2-trifluoro-acétyl)-1,2,3,4-tétrahydro-isoquinoline-7-sulfonique
ester tert-butylique de l'acide [2-phényl-1-(4-phényl-4-pipéridin-1-yl-cyclohexylcarbamoyl)-éthyl]-carbamique
ester benzylique de l'acide {[3-méthyl-1-(4-morpholin-4-yl-4-phényl-cyclohexylcarbamoyl)-butylcarbamoyl]-méthyl}-carbamique,
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement acceptables.

16. Procédé de fabrication de dérivés de cyclohexyl-1,4-diamine substitués selon l'une quelconque des revendications 1 à 10, **caractérisé en ce que** des cyclohexane-1,4-diamines substituées sont reliées avec des acides carboxyliques ou des acides sulfoniques de formule générale II 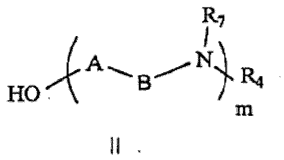 avec ajout de réactifs de couplage ou par activation du composant acide, notamment par fabrication du chlorure d'acide.

17. Médicament contenant au moins un dérivé de cyclohexyl-1,4-diamine substitué selon l'une quelconque des revendications 1 à 15, éventuellement sous la forme de son racémat, des stéréoisomères purs, notamment des énantiomères et des diastéréomères, en un rapport de mélange quelconque ; sous la forme de ses acides ou de ses bases ou sous la forme de ses sels, notamment des sels physiologiquement acceptables ou de sels d'acides ou de cations physiologiquement acceptables ; ou sous la forme de ses solvates, notamment des hydrates, et contenant éventuellement des additifs et/ou adjuvants appropriés et/ou éventuellement d'autres agents actifs.

18. Dérivés de cyclohexyl-1,4-diamine substitués selon l'une quelconque des revendications 1 à 15, éventuellement sous la forme de leurs racémats, des stéréoisomères purs, notamment des énantiomères et des diastéréomères, en un rapport de mélange quelconque ; sous la forme de leurs acides ou de leurs bases ou sous la forme de leurs sels, notamment des sels physiologiquement acceptables ou de sels d'acides ou de cations physiologiquement acceptables ; ou sous la forme de leurs solvates, notamment des hydrates, pour une utilisation pour le traitement de la douleur, notamment de la douleur aigue, neuropathique ou chronique, pour une utilisation pour le traitement des états d'anxiété, du stress et des syndromes associés avec le stress, des dépressions, de l'épilepsie, de la maladie d'Alzheimer, de la démence sénile, de la catalepsie, des dysfonctionnements cognitifs généraux, des difficultés d'apprentissage et de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse, l'anxiolyse, pour la modulation de l'activité motrice, pour la modulation de la distribution des neurotransmetteurs et le traitement des maladies neurodégénératives associées, pour le traitement des phénomènes de sevrage et/ou pour la réduction du potentiel d'accoutumance des opioïdes.
